(19)

**Europäisches Patentamt**
**European Patent Office**
**Office européen des brevets**

(11) **EP 3 916 001 A1**

(12) **EUROPEAN PATENT APPLICATION**
published in accordance with Art. 153(4) EPC

(43) Date of publication:
01.12.2021 Bulletin 2021/48

(21) Application number: 20745999.1

(22) Date of filing: 22.01.2020

(51) Int Cl.:
C07K 1/16 *(2006.01)*          C07K 1/18 *(2006.01)*
C07K 1/22 *(2006.01)*          C07K 1/36 *(2006.01)*
C07K 16/00 *(2006.01)*         C12P 21/08 *(2006.01)*

(86) International application number:
PCT/JP2020/002057

(87) International publication number:
WO 2020/153390 (30.07.2020 Gazette 2020/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.01.2019  JP 2019008865

(71) Applicant: Daiichi Sankyo Company, Limited
Tokyo 103-8426 (JP)

(72) Inventors:
• MASUDA, Yumiko
Tokyo 103-8426 (JP)

• OGINO, Yuka
Tokyo 103-8426 (JP)
• INOUE, Kota
Tokyo 103-8426 (JP)
• IZUMI, Kenta
Tokyo 103-8426 (JP)
• MUTO, Chie
Tokyo 103-8426 (JP)

(74) Representative: Fairbairn, Angus Chisholm
Marks & Clerk LLP
15 Fetter Lane
London EC4A 1BW (GB)

(54) **METHOD FOR ANTIBODY PURIFICATION INCLUDING STEP WHERE ACTIVATED CARBON MATERIAL IS USED**

(57)    To provide a method for purifying an antibody to a sufficient degree of purity for therapeutic use in humans by removing impurities contained in a solution efficiently while reducing production cost and the purification period in the purification of the antibody. It was found that an antibody can be reliably purified to a high degree of purity by an activated carbon material, regardless of the amounts or species of impurities co-present, and that high viral clearance can be attained reliably. Based on the finding, a treatment with an activated carbon material could be used in place of AEX chromatography achieving viral clearance in a step of purifying a therapeutic antibody using CHO cells. As a result, an antibody can be simply and effectively purified to a sufficient degree of purity for therapeutic use in humans compared to a conventional purification method, while reducing production cost.

**EP 3 916 001 A1**

**Description**

Technical Field

The present invention relates to a method for purifying an antibody to a high degree of purity.

Background Art

Recently, monoclonal antibodies have drawn significant attention and expectation as pharmaceuticals. Because of their specific binding ability to target antigens, antibodies are expected to have high efficacy and fewer side effects, and attract attention particularly as anti-cancer agents.

Many therapeutic antibodies are produced by production systems using CHO cells as hosts (Non-Patent Literature 1); however, cell culture supernatants contain not only the desired product, the antibody, but also host cell-derived impurities (e.g., protein, DNA), product-related impurities (e.g., aggregates, variants), and virus-like particles. It is known that since a host cell-derived protein (host cell protein; HCP) is recognized as a heterologous protein by humans, it is not only antigenic but also a tumorigenic risk. There is a concern that host cell-derived DNA is also tumorigenic. Aggregates, which are product-related impurities, may be immunogenic and fragments and desired proteins which have undergone post-translational modification have adverse effects on safety and efficacy. In addition, there is also a concern that endogenous viruses and endogenous virus-derived DNA are infectious. It is also necessary to take measures against contamination with exogenous viruses. In most cases, it is important to take measures in order to avoid risks of contamination with exogenous viruses from cell lines (for example, CHO cells mainly used in production), raw materials of culture media and prepared media, and to confirm absence of contamination with typical viruses likely mixed in production processes by tests such as in vitro and in vivo virus tests. Furthermore, it is necessary to reduce the risk of viral contamination of the finally obtained bulk drug as much as possible by evaluating the decrease and removal rates of typical model viruses in the purification process, and thereby, produce safe pharmaceuticals. For this reason, in the purification process, it is necessary to establish a purification method for effectively and reproducibly removing impurities while recovering an antibody (desired product) in a high yield.

An antibody purification method developed for the aforementioned purposes uses multiple steps of chromatography in combination. Affinity chromatography including protein A chromatography, ion exchange chromatography, hydrophobic interaction chromatography and ceramic hydroxyapatite chromatography are generally used. Host cell-derived impurities, product-related impurities, leached protein A and virus-like particles can be separated and removed efficiently by these chromatographies based on the charges, degrees of hydrophobicity or molecular sizes of the desired product and impurities, or the specific binding function between the desired product and an immobilized ligand.

Particularly, as a general chromatographic process for antibody purification, many literatures report a process for purifying an antibody to a high degree of purity by using three chromatographic steps: protein A chromatography, anion exchange (AEX) chromatography and cation exchange (CEX) chromatography, in combination. These chromatographies are performed generally in a bind/elution (B/E) mode or flow-through (F/T) mode. Of the modes, in protein A chromatography and CEX chromatography, the B/E mode in which an antibody (desired product) is captured onto, e.g., a resin, and then, impurities are separated under appropriate conditions is frequently used. Due to protein A chromatography and CEX chromatography performed in the B/E mode, a desired product can be highly purified except for the removal of viruses including endogenous viruses among the impurities. However, in the B/E mode, since the desired product is captured onto e.g., a resin, a large amount of resin and a large-size column are required, which increases production cost.

Meanwhile, with recent technological progress, a purification method of CEX chromatography by the F/T mode including an overload mode has been reported. An antibody (desired product) is allowed to pass through the column, while adsorbing impurities, the amount of which is lower than that of the product, onto, e.g., a resin. In this manner, the size of the column can be reduced and the operation process can be carried out more simply and efficiently, with the result that the amount of chromatography resin to be used can be reduced, attaining a cost reduction. Actually, in the B/E mode conventionally used, even if a high-performance resin is used, throughput was 100 g/L resin or less; whereas, in the F/T mode, even if the throughput was 1000 g/L resin or more, in other words, even if the amount of sample is increased 10-fold, a desired product can be purified. In other words, since the same purification performance can be achieved even if the amount of the resin is reduced to 1/10 or less, one can expect to reduce the size of the column, attaining the cost reduction. The column-size reduction results in a reduction of buffer volume to be used, tank sizes and workload. Such significant reductions contribute to improvement of production efficiency (reported in Patent Literature 1, Non-Patent Literature 2 and Non-Patent Literature 3). However, in the F/T mode of CEX chromatography, which is expected to attain a highly efficient purification step, since impurities co-present are separated/removed by adsorbing them onto, e.g., a small amount of resin, separation performance is affected by the amount and species of impurities co-present, unlike in the B/E mode, which poses a problem.

Furthermore, recently, productivity in the culture process has been improved. In accordance with the improvement, a large amount of cell culture products come to be loaded on a chromatography column, with the result that the load on the chromatography column increases. To deal with this problem, it is proposed that AEX chromatography and CEX chromatography performed in the F/T mode, which are expected as highly efficient purification steps, be used in combination with an activated carbon material, and it is reported that the amount of impurities including HCP is reduced, with the result that the lifetime of the chromatography column can be extended (Patent Literature 2).

However, these reports set forth only the possibility of removing general impurities (protein, DNA) derived from host cells and product-related impurities (e.g., aggregates, variants) derived from a desired product. Thus, it has been desired to develop a specific purification process, which realizes not only reliable removal of host cell-derived impurities and product-related impurities, which is desired as a basic function of the purification process, but also reliable removal of endogenous and exogenous viruses at low cost.

Citation List

Patent Literatures

> Patent Literature 1: International Publication No. WO2011/150110
> Patent Literature 2: International Publication No. WO2013/028330

Non-Patent Literatures

Non-Patent Literature 1: Reichert, J. (2012). Marketed therapeutic antibodies compendium. MABs 4:3, 413-415. Non-Patent Literature 2: Brown, A., Bill, J., Tully, T., Radhamohan, A., & Dowd, C. (2010). Overloading ionexchange membranes as a purification step for monoclonal antibodies. Biotechnol. Appl. Biochem., 56:59-70. Non-Patent Literature 3: Liu, H.F., McCooey, B., Duarte, T., Myers, D.E., Hudson, T., Amanullah, A., van Reis, R., & Kelley, B.D. (2011). Exploration of overloaded cation exchange chromatography for monoclonal antibody purification. J. Chromatogr. A., 1218, 6943-6952.

Summary of Invention

Technical Problem

The F/T mode of CEX chromatography, which is expected as a highly efficient purification step, had a problem in that separation performance is affected by the amount and species of impurities co-present, unlike the B/E mode. In an antibody purification method using AEX chromatography and CEX chromatography performed in the F/T mode in combination with an activated carbon material, the lifetime of the chromatography columns can be extended but with it a problem that the multiple purification steps are required. Put more specifically, since not only three chromatographic steps: protein A chromatography, AEX chromatography and CEX chromatography but also a treatment step with an activated carbon material are carried out, the multiple purification step increases production cost and the period of purification. In reducing the number of steps of a purification process to reduce the cost, there is a concern that not only performance to remove host cell-derived impurities and product-related impurities but also endogenous and exogenous viral clearance may decrease. Accordingly, reliable removal of impurities, particularly endogenous and exogenous viral clearance, is an important object. If the object is attained, not only a method for producing a therapeutic antibody at low cost and with high efficiency can be provided but also a high-quality therapeutic antibody can be stably supplied, with the result that safety of patients is ensured. For this reason, it is extremely important to attain this object, from an industrial point of view.

Solution to Problem

The present inventors conducted intensive studies with a view to attaining the object. As a result, they found an efficient purification method which reduces the number of purification steps while keeping comparable quality as in a conventional purification method. More specifically, they found that a desired antibody drug can be purified reliably to a high purity by CEX chromatography performed in the F/T mode, which is expected to be used at low cost but has a problem in that separation performance is low depending on the amount and species of impurities co-present, in combination with an activated carbon material by which the amount of impurities can be reduced. The present inventors also found that viruses can be removed by an activated carbon material reliably; and complementing the function of AEX chromatography in achieving viral clearance in the purification of therapeutic antibodies using CHO cells, with the result that a production process that can eliminate the step of AEX chromatography was developed. To summarize, the present inventors found an efficient purification method by using two chromatographic steps consisting of protein A chromatography and CEX chromatography performed in the F/T mode in combination with a treatment step with an activated carbon material. Due to this method, the number of purification steps and production cost can be reduced compared to a conventional purification method and an antibody can be purified efficiently to a sufficient quality for therapeutic use in humans. Based on this, the present invention was accomplished.

The present invention is summarized as follows.

[1] A method for purifying an antibody from a composition containing one or more impurities, comprising the steps of:

a1. providing a cell culture supernatant containing the antibody;
b1. processing the cell culture supernatant through an affinity chromatography resin to obtain an affinity chromatography pool containing the antibody;
c1. inactivating viruses in the affinity chromatography pool to obtain a virus inactivated solution;
d1. treating the virus inactivated solution with an activated carbon material to obtain an activated carbon material pool; and
e1. processing the activated carbon material pool through a cation exchange material to obtain a cation exchange material pool.

[2] The method for purifying an antibody from a composition containing one or more impurities according to [1], comprising the steps of:

a2. providing a cell culture supernatant containing the antibody;
b2. processing the cell culture supernatant through an affinity chromatography resin to obtain an affinity chromatography pool containing the antibody;
c2. inactivating viruses in the affinity chromatography pool to obtain a virus inactivated solution;
d2. processing the virus inactivated solution through a depth filter to obtain a depth filtration pool;
e2. treating the depth filtration pool with an activated carbon material to obtain an activated carbon material pool; and
f2. processing the activated carbon material pool through a cation exchange material to obtain a cation exchange material pool.

[3] The method according to [1] or [2], wherein the affinity chromatography resin is a protein A resin.
[4] The method according to [3], wherein the protein A resin is KanCapA, KanCapA3G, MabSelect SuRe, MabSelect SuRe pcc, MabSelect PrismA or Amsphere A3.
[5] The method according to any one of [1] to [4], wherein the virus inactivation is a treatment with at least one selected from the group consisting of an acid, a surfactant, a chemical substance, a nucleic acid crosslinking agent, ultraviolet rays, gamma rays and heat.
[6] The method according to [5], wherein the virus inactivation comprises reducing pH of the affinity chromatography pool to 3 to 4.
[7] The method according to [6], wherein the affinity chromatography pool is incubated for 60 to 120 minutes during the virus inactivation.
[8] The method according to [6] or [7], wherein the affinity chromatography pool is incubated at 15 to 30°C during the virus inactivation.
[9] The method according to any one of [1] to [8], wherein the solution to be loaded in the activated carbon material, and an equilibration buffer and a wash buffer for the activated carbon material each have a pH of 4.5 to 7.5.
[10] The method according to [9], wherein the solution to be loaded in the activated carbon material, and the equilibration buffer and the wash buffer for the activated carbon material each have a pH of 4.5 to 5.5.
[11] The method according to any one of [1] to [10], wherein the solution to be loaded in the activated carbon material, and an equilibration buffer and a wash buffer for the activated carbon material each have an electrical conductivity of 2 to 10 mS/cm.
[12] The method according to [11], wherein the solution to be loaded in the activated carbon material, and the equilibration buffer and the wash buffer for the activated carbon material each have an electrical conductivity of 2 to 5 mS/cm.
[13] The method according to any one of [1] to [12], wherein the treatment with an activated carbon material is carried out by use of a filter containing the activated carbon material.
[14] The method according to [13], wherein the filter is Millistak+ Pod CR40, Zeta Plus activated carbon adsorption depth filter or SUPRAcap 50 Seitz AKS filter.

[15] The method according to any one of [1] to [14], wherein the activated carbon material pool is loaded in the cation exchange material in an amount exceeding the binding capacity of the cation exchange material.

[16] The method according to [15], wherein the activated carbon material pool is loaded in the cation exchange material at a loading density of 500 to 2000 g/L.

[17] The method according to [16], wherein the activated carbon material pool is loaded in the cation exchange material at a loading density of 1000 to 1500 g/L.

[18] The method according to any one of [1] to [17], wherein the cation exchange material is a resin, a monolith column or a membrane.

[19] The method according to [18], wherein the cation exchange material has a functional group, which is sulfopropyl, sulfoethyl, sulfoisobutyl or carboxyl.

[20] The method according to [19], wherein the cation exchange material is POROS XS, Eshmuno CPX, Eshmuno CP-FT or CaptoS ImpAct.

[21] The method according to any one of [1] to [20], wherein the solution to be loaded in the cation exchange material has a pH of 4 to 6.

[22] The method according to [21], wherein the solution to be loaded in the cation exchange material has a pH of 4.5 to 5.5.

[23] The method according to any one of [1] to [22], wherein the solution to be loaded in the cation exchange material has an electrical conductivity of 3 to 7 mS/cm.

[24] The method according to [23], wherein the solution to be loaded in the cation exchange material has an electrical conductivity of 3 to 5 mS/cm.

[25] The method according to any one of [1] to [24], wherein the cation exchange material pool is further processed through hydrophobic interaction chromatography to obtain a pool solution.

[26] The method according to [25], wherein the hydrophobic interaction chromatography is performed in a flow-through mode.

[27] The method according to [26], wherein the hydrophobic interaction chromatography is performed by use of a resin or a membrane.

[28] The method according to [27], wherein the hydrophobic interaction chromatography resin or membrane has a phenyl group, a benzyl group or a hexyl group.

[29] The method according to [28], wherein the hydrophobic interaction chromatography material is POROS Benzyl Ultra, POROS Benzyl, Hexyl-650C, Phenyl Sepharose 6 Fast Flow, Phenyl Sepharose High Performance, Sartobind Phenyl or ReadyToProcess Adsorber Phenyl.

[30] The method according to any one of [1] to [14], wherein the activated carbon material pool is processed through hydrophobic interaction chromatography to obtain a pool solution, which is further processed through a cation exchange material to obtain a pool solution.

[31] The method according to [30], wherein the hydrophobic interaction chromatography is performed in a flow-through mode.

[32] The method according to [31], wherein the hydrophobic interaction chromatography is performed by use of a resin or a membrane.

[33] The method according to [32], wherein the hydrophobic interaction chromatography resin or membrane has a phenyl group, a benzyl group or a hexyl group.

[34] The method according to [33], wherein the hydrophobic interaction chromatography material is POROS Benzyl Ultra, POROS Benzyl, Hexyl-650C, Phenyl Sepharose 6 Fast Flow, Phenyl Sepharose High Performance, Sartobind Phenyl or ReadyToProcess Adsorber Phenyl.

[35] The method according to any one of [30] to [34], wherein the hydrophobic interaction chromatography pool is loaded in the cation exchange material in an amount exceeding the binding capacity of the cation exchange material.

[36] The method according to [35], wherein the hydrophobic interaction chromatography pool is loaded in the cation exchange material at a loading density of 500 to 2000 g/L.

[37] The method according to [36], wherein the hydrophobic interaction chromatography pool is loaded in the cation exchange material at a loading density of 1000 to 1500 g/L.

[38] The method according to any one of [30] to [37], wherein the cation exchange material is a resin, a monolith column or a membrane.

[39] The method according to [38], wherein the cation exchange material has a functional group, which is sulfopropyl, sulfoethyl, sulfoisobutyl or carboxyl.

[40] The method according to [39], wherein the cation exchange material is POROS XS, Eshmuno CPX, Eshmuno CP-FT or CaptoS ImpAct.

[41] The method according to any one of [30] to [40], wherein the solution to be loaded in the cation exchange material has a pH of 4 to 6.

[42] The method according to [41], wherein the solution to be loaded in the cation exchange material has a pH of 4.5 to 5.5.

[43] The method according to any one of [30] to [42], wherein the solution to be loaded in the cation exchange material has an electrical conductivity of 3 to 7 mS/cm.

[44] The method according to [43], wherein the solution to be loaded in the cation exchange material has an electrical conductivity of 3 to 5 mS/cm.

[45] The method according to any one of [1] to [44], wherein at least one of the impurities is selected from the group consisting of host cell-derived proteins (host cell proteins; HCPs), phospholipase B-like 2 (PLBL2), viruses, virus-like particles, high-molecular weight species (HMWS), low-molecular weight species (LMWS), culture medium components, leached protein A and/or DNA.

[46] The method according to any one of [1] to [45], wherein the steps are carried out continuously.

[47] An antibody purified by the method according to any one of [1] to [46].

[48] A method for purifying an antibody from a composition containing one or more impurities, comprising the steps of:

    a1. providing a cell culture supernatant containing the antibody;
    b1. processing the cell culture supernatant through an affinity chromatography resin to obtain an affinity chromatography pool containing the antibody;
    c1. inactivating viruses in the affinity chromatography pool to obtain a virus inactivated solution; and
    d1. treating the virus inactivated solution with an activated carbon material to obtain an activated carbon material pool,
    wherein PLBL2 clearance performance is improved by carrying out step d1.

[49] A method for purifying an antibody from a composition containing one or more impurities, comprising the steps of:

    a2. providing a cell culture supernatant containing the antibody;
    b2. processing the cell culture supernatant through an affinity chromatography resin to obtain an affinity chromatography pool containing the antibody;
    c2. inactivating viruses in the affinity chromatography pool to obtain a virus inactivated solution;
    d2. treating the virus inactivated solution with a depth filter to obtain a depth filtration pool; and
    e2. treating the depth filtration pool with an activated carbon material to obtain an activated carbon material pool,
    wherein PLBL2 clearance performance is improved by carrying out step e2.

[50] A method for purifying an antibody from a composition containing one or more impurities, comprising the steps of:

    a1. providing a cell culture supernatant containing the antibody;
    b1. processing the cell culture supernatant through an affinity chromatography resin to obtain an affinity chromatography pool containing the antibody;
    c1. inactivating viruses in the affinity chromatography pool to obtain a virus inactivated solution;
    d1. treating the virus inactivated solution with an activated carbon material to obtain an activated carbon material pool; and
    e1. processing the activated carbon material pool through a cation exchange material to obtain a cation exchange material pool,
    wherein PLBL2 clearance performance is improved by carrying out step d1.

[51] A method for purifying an antibody from a composition containing one or more impurities, comprising the steps of:

    a2. providing a cell culture supernatant containing the antibody;
    b2. processing the cell culture supernatant through an affinity chromatography resin to obtain an affinity chromatography pool containing the antibody;
    c2.inactivating viruses in the affinity chromatography pool to obtain a virus inactivated solution;
    d2. processing the virus inactivated solution through a depth filter to obtain a depth filtration pool;
    e2. treating the depth filtration pool with an activated carbon material to obtain an activated carbon material pool; and
    f2. processing the activated carbon material pool through a cation exchange material to obtain a cation exchange material pool,
    wherein PLBL2 clearance performance is improved by carrying out step e2.

Advantageous Effects of Invention

The present inventors added a treatment with an activated carbon material to a conventional antibody purification process, and found that impurity load on a subsequent purification step can be reduced and an antibody can be reliably purified to a desired degree of purity, regardless of the amount and species of impurities co-present. The present inventors further found that a high viral clearance performance can be achieved by the treatment with an activated carbon material, complementing the function of AEX chromatography achieving viral clearance in purification of antibody drugs using CHO cells, with the result that a production process that can eliminate AEX chromatography was developed. Due to the production process, they succeeded in purifying an antibody to a sufficient degree of purification for therapeutic use in humans with a reduction in the number of purification steps and production cost, compared to a conventional purification method.

Description of Embodiments

Now, the present invention will be more specifically described.

1. Definition

In the specification, the term "antibody" refers to, e.g., a monoclonal antibody, a polyclonal antibody, a chimeric antibody, a humanized antibody, a human antibody, a multi-specific antibody (bispecific antibody) formed of at least two intact antibodies, an Fc fusion protein and an antibody fragment having a biological activity. Note that, the antibody to which the present invention can be applied is not limited by an antigen to which the antibody is to be specifically bound. Also the antibody to which the present invention can be applied is not limited by the amino acid sequence of the antibody.

In the specification, the term "bind/elute (B/E) mode" refers to a technical manner for separating a product by binding at least one product contained in a sample to a chromatography resin or medium and thereafter eluting the product.

In the specification, the term "flow-through (F/T) mode", which implies that at least one product contained in a sample flows through a chromatography resin or medium, refers to a technical manner for separating a product by binding at least one latent component to a chromatography resin or medium.

In the specification, the term "overload mode", which implies that loading is carried out in an amount exceeding the binding capacity of a column or the like, refers to a technical manner for separating a product by using a difference in the amount of adsorption between a desired product and impurities. In the specification, a partially purified product of an antibody is loaded in a cation exchange material in an amount exceeding the binding capacity of the cation exchange material. The binding capacity of the partially purified product of an antibody to a cation exchange material is about 100 g in terms of protein per cation exchange material (1 L). The loading density in the overload mode is 150 to 4000 g in terms of protein per cation exchange material (1 L). A preferable loading density falls within the range of 500 to 2000 g/L and a further preferable loading density falls within the range of 1000 to 1500 g/L.

In the specification, the term "affinity chromatography" refers to a technical method for separating a protein by binding a target protein (for example, a protein or antibody having a desired Fc region) specifically to a ligand specific to the target protein. When an antibody is purified, such a ligand is protein A, protein G or a functional variant thereof. The ligand is allowed to covalently bind to a solid-phase material of a chromatography column and binds to an antibody when a solution containing the antibody is in contact with the solid-phase material of the chromatography column.

In the specification, the term "depth filter" refers to a set of filters, which are used in removing particles from an antibody solution and serially arranged, which have decreasing pore size. The depth filter has a three-dimensional matrix having a maze-like passage through which the antibody solution flows. Particles are captured onto and mechanically trapped at random by the overall matrix to the bottom. The depth filters are composed of a wound cotton, a polypropylene, a rayon cellulose, a glass fiber, a sintered metal, porcelain, diatomaceous earth or a known component other than these.

In the specification, the term "cation exchange material" and the term "CEX material" are interchangeably used and each refer to a solid phase negatively charged and thus having free cations for exchanging with cations in an aqueous solution passed on or through the solid phase.

In the specification, the term "anion exchange material" and the term "AEX material" are interchangeably used and each refer to a positively charged solid phase having a single type or a plurality of types of positively charged ligands bound to the solid phase, such as a primary amino group, a secondary amino group, a tertiary amino group or a quaternary amino group.

In the specification, the term "hydrophobic interaction chromatography" or "HIC" refers to a process for separating a molecule based on hydrophobicity of the molecule, in other words, the ability of molecule to adsorb onto a hydrophobic surface from an aqueous solution. The hydrophobic interaction chromatography generally functions depending on difference in type of hydrophobic groups on the surface of a solute molecule. Hydrophobic groups have a tendency of binding to hydrophobic groups on the surface of an insoluble matrix. HIC has a higher polarity than reversed-phase liquid chromatography and is employed in an environment of low degeneration. HIC is thus generally used for antibody purification often in combination with ion exchange or gel filtration chromatography.

In the specification, the term "activated carbon material" refers to any substance as long as it is constituted of or contains carbon, or activated carbon. The "activated carbon" refers to a carbonaceous material which has been subjected to a process to enhance its pore structure. Activated carbon is a porous solid having an extremely high surface area. Activated carbon can be obtained from a wide variety of supply sources including coal, woods, coconut husk, nutshells and peat. Activated carbon can be produced from these materials by physical activation including application of heat in a controlled environment or chemical activation using a strong acid, base or an oxidant. According to these activation methods, a porous structure having a large surface area, which provides a high ability to remove impurities to activated carbon, can be produced. Activated carbon, unlike most of other adsorption materials, is considered to interact with molecules by means of relatively weak van der Waals force. As the activated carbon to be used in the purification method of the present invention, a single type of activated carbon can be used alone or two types or more activated carbons can be used alone or in mixture.

In the specification, the term "continuously" means having any other means which enables continuous flow between materials directly connected. More specifically, the term means a process for purifying a desired product and including two or more process steps (or unit operations) which allow output from one process step to flow directly into the next process step without intervention, and which can be at least partly and simultaneously operated during the period.

In the specification, the terms "removal", "reduction" and "clearance" are interchangeably used and each refer to reducing the amount of one or more impurities in a sample containing the antibody to be purified.

In the specification, the terms "purification" and "increase a degree of purity" are interchangeably used and each means that the ratio of a target antibody relative to at least one species of impurity in a sample is increased by removing the impurity(ies) from the sample.

In the specification, the term "electrical conductivity" refers to an ability of an aqueous solution to transmit electric current between two electrodes. In an aqueous solution, electric current flows by ion transport. Accordingly, if the amount of ions present in an aqueous solution increases, the electrical conductivity of the solution increases. Electrical conductivity can be measured by an electrical conductivity detector and the measurement unit of electrical conductivity is milli-siemens per centimeter (mS/cm or mS). The electrical conductivity of a solution can be changed by changing the concentration of ions in the solution. For example, in the concentration of a buffering agent and/or a salt in a solution can be changed in order to attain a desired electrical conductivity. Preferably, the salt concentrations of buffer solutions can be changed in order to obtain a desired electrical conductivity.

In the specification, the term "fraction" refers to a small volume of a fluid collected after a sample containing a target antibody is subjected to a step of loading the sample in an activated carbon material, for example.

In the specification, the term "HCP; host cell protein" refers to a mixture of host cell-derived proteins derived from a Chinese hamster ovary (CHO) cell culture. HCP is generally present as impurities in a cell culture containing a desired protein such as an antibody expressed in CHO cells.

In the specification, the term "HMWS; high-molecular weight species" refers to one of the antibody variants different in molecular weight that elutes in a higher molecular weight region than a monomer.

In the specification, the term "LMWS; low-molecular weight species" refers to one of the antibody variants different in molecular weight that elutes in a lower molecular weight region than a monomer.

In the specification, the term "virus" refers to a small infectious agent or particle replicable only within living cells. The viruses each generally consist of a nucleic acid (RNA or DNA) enclosed in a protein capsid, which may be optionally a lipid envelope.

In the specification, the term "virus-like particles" refers to a structure observed by an electron microscope and conceivably having a morphological relationship with a known virus.

In the specification, the term "retrovirus" refers to an RNA virus, whose genetic information can be integrated into genomic DNA of a cell infected with the RNA virus via a reverse-transcribed DNA intermediate.

In the specification, the term "retrovirus-like particles" refers to particles produced by the cell having retrovirus-derived DNA portions in the genome. The retrovirus-like particles resemble retroviruses but they are mostly non-infectious. Murine leukemia virus (MLV) used in Examples of the specification represents a model retrovirus or a retrovirus-like particle.

In the specification, the term "parvovirus" refers to a linear, non-segmented single-stranded DNA virus with no envelope, having an average genome size of 5000 nucleotides and a diameter of 18 to 26 nm. A parvovirus generally used in the method described in the specification is murine minute virus (MMV).

In the specification, the "log reduction value" or "LRV" refers to a value representing a performance of a column or a filter to remove impurities and is obtained by passing impurities known in amount through the column or the filter.

$$LRV = \log(Cf) - \log(Cp)$$

where Cf represents the concentration of impurities in a loading solution; and Cp represents the concentration of residual impurities in the solution passed through a column or a filter.

In the specification, examples of the "culture medium component" include serum, a growth factor (e.g., insulin) and antibiotic substance.

2. Removal of HCP by CEX chromatography in the F/T mode

An antibody cell culture was purified by protein A chromatography, AEX chromatography and CEX chromatography in the F/T mode successively in this order in accordance with conventional techniques shown in Patent Literature 1, Non-Patent Literature 2 and Non-Patent Literature 3. As a result, it was found that HCP cannot be sufficiently removed in some cases depending on the types of antibody (including differences in the amount and/or species of impurities co-present due to differences in host cell and culture method). A process consisting of protein A chromatography, AEX chromatography and CEX chromatography in the F/T mode, which are subsequently carried out in this order, was excellent in view of cost, labor and operation time; however, it was found that removability of impurities, particularly HCP, is not reliable.

It has been widely known that HCP can induce immunogenicity via various mechanisms. Actually, in clinical trials of a biosimilar of somatropin, it was reported that an anti-HCP antibody was produced by residual HCP in all patients, and that a non-neutralizing anti-somatropin antibody was produced in at most 60% of the patients (M. Pavlovic et al., Horm. Res. 69:14-21 (2008)). The level of the anti-somatropin antibody was improved by further removing HCP to the same level as those reported in the cases of other growth hormone antibodies. In the clinical trial of a recombinant IX factor produced in CHO cells, it was reported that an anti-HCP antibody was produced at a considerably high percentage; and that although adverse events were not found, a clinical hold order was issued from the regulatory authority (https://www.fda.gov/downloads/BiologicsBloodVaccines/Blo odBloodProducts/ApprovedProducts/LicensedProducts-BLAs/Fra ctionatedPlasmaProducts/UCM448429.pdf Food and Drug Administration STATISTICAL REVIEW AND EVALUATION BLA FINAL, Product Name: IB1001, Indication(s): Control and prevention of bleeding episodes and perioperative management in patients with hemophilia B, CBER Receipt Date: Apr 6 2012 or https://www.news-medical.net/news/20120710/Inspiration-receives-FDA-clinical-hold-order-for-IB1001-phase-III-studies-on-hemophilia-B.aspx News-Medical.net - An AZoNetwork Site Inspiration receives FDA clinical hold order for IB1001 phase III studies on hemophilia B, Jul 10 2012).

Regardless of the presence or absence of an adverse event report on safety, the regulatory authority is concerned about a potential risk and may give instructions to carry out clinical trials after the amount of residual HCP is further reduced. Recently, it has been reported that polysorbate in a drug product is degraded by a residual HCP (lipoprotein lipase) and this affects the stability of pharmaceuticals (T Hall et al., J. Chromatogr. A 105: 1633-1642 (2016)). In view of the stability of pharmaceuticals, it is important to appropriately remove HCP in a purification step.

The effects of residual HCP on safety and stability conceivably vary depending on not only the type of host cell and the species of residual HCP but also the dosage and administration of a product. Accordingly, the acceptable value of residual HCP must be appropriately set for every product in consideration of e.g., the production process, maximum dose, administration site and administration frequency; however, it is desired to reduce residual HCP as much as possible in the production process.

As mentioned above, also in a purification flow using CEX chromatography in the F/T mode, it has been desired to develop a method for reducing impurities including HCP generally applied to any type of antibody.

3. Removal of impurities by application of activated carbon material

The present inventors conducted studies with a view to developing a method for purifying an antibody to a high degree of purity reliably while reducing production cost. As a result, they found that any types of antibody can be purified to a high degree of purity by adding a treatment with an activated carbon material to a conventional purification step.

As described above, HCP is required to be removed to a sufficiently low level by purification steps, in consideration that HCP can be antigenic as heterologous protein against humans; a mammalian cell-derived protein has a risk of tumorigenicity; and HCP is concerned with the stability of pharmaceuticals by degradation of a polysorbate in pharmaceuticals. Phospholipase B-like 2 (PLBL2), which is a kind of HCP, is reported as hitchhiker HCP, which remains through interaction with an antibody (B. Tran et al., J. Chromatogr. A 1438: 31-38 (2016)). There is a report that an anti-hamster PLBL2 antibody is very frequently generated by residual PLBL2 in drug products in clinical trials of lebrikizumab (International Publication No. WO2015/038888). A clinical significance of the generation of an anti-hamster PLBL2 antibody has not been found; and there was no correlation between the generation of the anti-hamster PLBL2 antibody even though it occurs very frequently and a safety event, in the clinical trials of lebrikizumab. Nevertheless, there is still a risk that long-term exposure by repeated administration may increase undesirable effects such as anaphylaxis and hypersensitivity in an allergic or hypersensitive patient population. Because of this, it is considered desirable that the level of PLBL2 in drug products is reduced as much as possible.

To satisfy these desires, a treatment with an activated carbon material was added. Due to the treatment, the impurities loaded on CEX chromatography were reduced, with the result that HCP was successfully reduced to a sufficiently low level. Additionally, it is difficult to reduce the amount of PLBL2 (hitchhiker HCP), which remains due to interaction with an antibody, by a general purification method. For this reason, reduction of PLBL2 by about 50% in load by an activated carbon material, in other words, obtaining a PLBL2 log reduction value (LRV) of 0.27 by an activated carbon material, has great significance.

Furthermore, product-related impurities, HMWS and LMWS, were reduced by an activated carbon material. A reduction of HMWS by an activated carbon material can reduce the load on CEX chromatography, with the result that the amount of HMWS in a finally purified product can be reliably reduced. Since it is difficult to reduce the amount of LMWS by a general purification method, the contribution of an activated carbon material to the purity increase of a finally purified product is high.

In addition to these, the pH of an antibody solution to be loaded on an activated carbon material was adjusted, with the result that viral clearance performance was improved. It is shown that retroviruses and/or retrovirus-like particles can be removed by an activated carbon material, or that parvoviruses can be removed by an activated carbon material, although the removal effect is limited depending on the antibody solution (International Publication No. WO2015/005961); in this case, the pH of the solution is 7.0. However, in the present invention, it was found that viruses can be removed under the condition of pH 5.0, and it was further demonstrated that viral clearance performance under the condition of pH 5.0 is better than that under the conditions disclosed in previous reports.

A general antibody purification method is carried out by protein A chromatography and virus inactivation treatment at low pH. Following the virus inactivation treatment, pH is sometimes neutralized to about pH 5.0. In the following CEX chromatography, the condition of about pH 5.0 is frequently employed. From this, if a treatment with an activated carbon material is carried out between these steps, to carry out the treatment with an activated carbon material without specific operations such as pH adjustment and dilution greatly contributes to simplification of production operation. Furthermore, an antibody is sometimes destabilized by pH change depending on the type of antibody. Because of this, it is considered that a treatment with an activated carbon material without pH adjustment greatly contributes to keeping the stability of an antibody.

In a purification process of a therapeutic antibody using CHO cells, it is generally known that AEX chromatography achieves viral clearance. Since it was found that an activated carbon material also achieves the same level of viral clearance as AEX chromatography, a treatment step with an activated carbon material can be used in place of the AEX chromatography. Thus, the possibility of eliminating AEX chromatography was found. Chromatography including AEX chromatography is complicated in operation. If an activated carbon material is used as an adsorption filter, chromatography complicated in operation can be eliminated, with the result that the purification process can be more simple and effective. Furthermore, since an expensive AEX resin or Membrane Adsorber are replaced with an inexpensive activated carbon adsorption filter, a running cost can be significantly reduced. Moreover, since expensive chromatography equipment is not required and an inexpensive filter unit can be used instead, it is considered that the initial investment for a production facility can be greatly reduced.

4. Purification step employed in the present invention

In the specification, a method (hereinafter referred to as improved method 2) for purifying an antibody from a composition containing one or more impurities is provided, which comprises the steps of:

a1. providing a cell culture supernatant containing the antibody;
b1. processing the cell culture supernatant through an affinity chromatography resin to obtain an affinity chromatography pool containing the antibody;
c1. inactivating viruses in the affinity chromatography pool to obtain a virus inactivated solution;
d1. treating the virus inactivated solution with an activated carbon material to obtain an activated carbon material pool; and
e1. processing the activated carbon material pool through a cation exchange material to obtain a cation exchange material pool.

Preferable examples of the affinity chromatography resin in step b1 include a protein A resin. Examples of protein A resin that can be employed include, but are not limited to, KanCapA (KANEKA Corporation), KanCapA3G (KANEKA Corporation), MabSelect SuRe (GE Healthcare Life Science), MabSelect SuRe pcc (GE Healthcare Life Science), MabSelect PrismA (GE Healthcare Life Science) and Amsphere A3 (JSR Life Sciences Corporation).

The virus inactivation in step c1 can be carried out by one method selected from treatments with an acid, a surfactant, a chemical substance, a nucleic acid crosslinking agent, ultraviolet rays, gamma rays and heat. If a treatment with an acid is selected for inactivating viruses, the treatment to lower the pH of an affinity chromatography pool to the range 3 to 4 is preferable, and further preferable pH of the treatment for inactivating viruses is 3.5. In this case, a preferable treatment time is 60 to 120 minutes and a preferable treatment temperature is 15 to 30°C.

In step d1, an activated carbon material is packed in a chromatography column. According to another embodiment, an activated carbon material is a filter-type and placed in a closed disposable device such as Millistak+ (R) Pod. According to yet another embodiment, an activated carbon material is placed in a cartridge or a capsule. In most cases, a filter-type activated carbon material is prepared by mixing an activated carbon material with a stiff filter-material such as a cellulose matrix.

Examples of a commercially available activated carbon filter include, but are not limited to, Millistak+ (R) Pod CR40 (Merck Millipore), Zeta Plus activated carbon adsorption depth filter (3M Company) and SUPRAcap 50 Seitz (R) AKS filter (PALL Corporation). In activated carbon filters according to some embodiments, an activated carbon material is held in a cellulose matrix. In step d1, the pH of the solution to be loaded on the activated carbon material, and an equilibration buffer and a wash buffer for the activated carbon material is preferably 4.5 to 7.5, more preferably 4.5 to 5.5 and further preferably 5.0. In step d1, the electrical conductivity of the solution to be loaded on the activated carbon material, and an equilibration buffer and a wash buffer for the activated carbon material is preferably 2 to 10 mS/cm and more preferably 2 to 5 mS/cm.

In step e1, as the cation exchange material, any one of a resin, a monolith column and a membrane is selected. The functional group of the cation exchange material is sulfopropyl, sulfoethyl, sulfoisobutyl, or carboxyl. Examples of a commercially available cation exchange material include, but are not limited to, POROS XS (Thermo Fisher Scientific Inc.), Eshmuno (R) CPX (Merck Millipore), Eshmuno (R) CP-FT (Merck Millipore) and Capto S ImpAct (GE Healthcare Life Science).

In step e1, the activated carbon material pool obtained in step d1 is loaded on the cation exchange material in an amount exceeding the binding capacity. The loading density can be selected within the range of 150 to 4000 g of protein amount per 1 L of cation exchange material, preferably falls within the range of 500 to 2000 g/L, and further preferably 1000 to 1500 g/L. The pH of the activated carbon material pool to be loaded is preferably 4 to 6, more preferably 4.5 to 5.5 and further preferably 5.0. In step e1, the electrical conductivity of the solution to be loaded on the cation exchange material is preferably 3 to 7 mS/cm and more preferably 3 to 5 mS/cm.

In improved method 2, at least one step may be optionally added before and after each of steps a1 to e1.

For example, a processing step through a depth filter may be added between step c1 and step d1 of improved method 2. The depth filter is a filter, which can trap particles having a size larger than about 1 μm by use of two actions, i.e., a capture action and a mechanical filtration action of an ordinary porous filter material, and which is formed of a filter material such as cellulose matrix and diatomaceous earth. The depth filter has a characteristic of trapping objects by a thick filter material having numerous long flow channels. The depth filter is placed in a closed disposable device such as Millistak+ (R) Pod. Examples of a commercially available depth filter include, but are not limited to, Millistak+ (R) A1HC, D0HC and X0HC.

As a specific method further comprising a processing step through a depth filter, there is provided a method for purifying an antibody from a composition containing one or more impurities, comprising the steps of:

a2. providing a cell culture supernatant containing the antibody;
b2. processing the cell culture supernatant through an affinity chromatography resin to obtain an affinity chromatography pool containing the antibody;
c2.inactivating viruses in the affinity chromatography pool to obtain a virus inactivated solution;
d2. processing the virus inactivated solution through a depth filter to obtain a depth filtration pool;
e2. treating the depth filtration pool with an activated carbon material to obtain an activated carbon material pool; and
f2. processing the activated carbon material pool through a cation exchange material to obtain a cation exchange material pool. This is a method (hereinafter referred to as improved method 1) in which a processing step through a depth filter is added between step c1 and step d1 of improved method 2.

A processing step through an anion exchange material may be added between step c1 and step d1 of improved method 2 or between step d2 and step e2 of improved method 1. As the anion exchange material, any one of a resin, a monolith column and a membrane is selected. As the membrane, Q membrane can be selected. Ordinarily, the anion exchange material is used in the F/T mode. Examples of a commercially available anion exchange material include, but are not limited to, NatriFlo (R) HD-Q, Mustang (R) Q and Sartobind (R) Q.

The cation exchange material pool obtained in step e1 of improved method 2 or step f2 of improved method 1 may be further processed through hydrophobic interaction chromatography to obtain a pool solution (hereinafter referred to as improved method 3). The activated carbon material pool obtained in step d1 of improved method 2 or step e2 of improved method 1 is further processed through hydrophobic interaction chromatography to obtain a hydrophobic interaction chromatography pool, which may be further processed through a cation exchange material to obtain a cation exchange material pool (hereinafter referred to as improved method 4). The hydrophobic interaction chromatography is preferably performed in the F/T mode. The hydrophobic interaction chromatography is performed by use of a resin or a membrane having a phenyl group, a benzyl group or a hexyl group. Examples of a commercially available material for hydrophobic interaction chromatography include, but are not limited to, Phenyl Sepharose High Performance (GE Healthcare Life Science), Phenyl Sepharose 6 Fast Flow (GE Healthcare Life Science), Sartobind (R) Phenyl (sartorius), ReadyToProcess Adsorber Phenyl (GE Healthcare Life Science), POROS Benzyl Ultra (Thermo Fisher Scientific Inc.), POROS Benzyl (Thermo Fisher Scientific Inc.) and Hexyl-650C (Tosoh Corporation).

According to some embodiments of any one of the methods described therein, a step of recovering an antibody purified is further included. According to some embodiments, a purified antibody is recovered from any one of the purification steps described therein.

At least one species of impurity selected from HCP, PLBL2, virus, virus-like particles, HMWS, LMWS, culture medium component, leached protein A and DNA is removed by the steps described above.

Examples

The present invention will be more specifically described by way of the following Examples; however, the present invention is not limited by these Examples.

Example 1: Methods used in common In this Example, methods, which are used in common in Example 2 and subsequent Examples, will be described.

1) Measurement of antibody concentration

Antibody concentration was measured by HPLC using PA ID Sensor Cartridge (2.1 mm × 3 mm, Applied Biosystems) column.

2) HCP measurement

HCP was measured by use of third-generation CHO host cell protein kit from Cygnus Technologies or CHO|360 HCP ELISA kit from BioGENES GmbH in accordance with enzyme-linked immunosorbent assay (ELISA). The HCP concentration obtained was converted to a value ng HCP/mg antibody by dividing it by the antibody concentration.

In Examples 2 to 5, the third-generation CHO host cell protein kit from Cygnus Technologies was used; whereas in Examples 6 to 8, CHO|360 HCP ELISA kit from BioGENES GmbH was used.

3) PLBL2 measurement

PLBL2 was measured by use of Hamster Phospholipase B-Like 2 (PLBL2) ELISA kit from Immunology Consultants Laboratory, INC. in accordance with enzyme-linked immunosorbent assay (ELISA). The PLBL2 concentration obtained was converted to a value ng PLBL2/mg antibody by dividing it by the antibody concentration. In each purification step, PLBL2 clearance performance (log reduction value; LRV) was calculated in accordance with the following formula:

$$\mathrm{LRV} = \log(\mathrm{Cf}) - \log(\mathrm{Cp})$$

where Cf represents the concentration of PLBL2 in a loading solution; and Cp represents the concentration of residual PLBL2 in the solution passed through a column or a filter.

4) Contents of high-molecular weight species (HMWS), low-molecular weight species (LMWS) and monomer

Measurements were conducted by HPLC using TSK gel G3000SWXL column (5 μm, 7.8 mm × 300 mm, Tosoh Corporation) or ACQUITY UPLC Protein BEH SEC column (1.7 μm, 4.6 mm × 300 mm, Waters Corporation). A 0.1 M phosphate buffer containing 0.4 M sodium chloride was used as the mobile phase.

5) Measurement of protein A

Protein A was measured by use of protein A ELISA kit from Cygnus Technologies in accordance with enzyme-linked immunosorbent assay (ELISA). The protein A concentration obtained was converted to a value ng protein A/mg antibody by dividing it by the antibody concentration in order to compare the results.

6) Measurement of virus titer and calculation of viral clearance performance (log reduction value; LRV)

Virus titer was measured by a method (median tissue culture infections dose; $TCID_{50}$) determining the viral load which infects 50% of cells based on the phenomenon (cytopathy) where the shape of cells changes when they are infected with viruses. The viruses subjected to the measurement were murine leukemia virus (MLV), murine minute virus (MMV), pseudorabies virus (PRV) and reovirus type 3 (Reo3). The indicator cells used were PG4 cells (indicator cells of MLV), 324K cells or A9 cells (indicator cells of MMV), Vero cells (indicator cells of PRV) and L-929 cells (indicator cells of Reo3). Prior to a viral clearance study, the buffer solution and samples were examined as to whether they have undesirable effects on indicator cells based on toxicity or interference to a virus titer assay. Viral clearance performance (log reduction value; LRV) was calculated in accordance with the following formula:

$$LRV = \log(Cf) - \log(Cp)$$

where Cf represents the concentration of viruses in a loading solution; and Cp represents the concentration of remaining viruses in the solution passed through a column or a filter.

7) Measurement of DNA

The concentration of DNA was measured by qPCR method using primers and a probe specific to CHO. The DNA concentration obtained was converted to a value pg DNA/mg antibody by dividing it by the antibody concentration.

Example 2: Reference Example: Purification by CEX chromatography in F/T mode (Mab A)

In this Example, clearance of impurities in a purification flow of a monoclonal antibody referred to as Mab A will be described in accordance with the flowchart shown in Table 1 below.

[Table 1]

| Clarification of cell culture |
| --- |
| ↓ |
| Affinity chromatography |
| ↓ |
| Virus inactivation |
| ↓ |
| Depth filtration |
| ↓ |
| AEX chromatography |
| ↓ |
| CEX chromatography (F/T mode) |

1) Materials and methods

The cell culture containing Mab A produced by CHO cells was filtered with a depth filter to remove the cells and subjected to clarification to obtain a cell culture supernatant. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab A was eluted from the column with an acetate buffer (pH 3.6). The absorbance at 280 mm was monitored and fractions containing Mab A were obtained (affinity chromatography pool). To the affinity chromatography pool, hydrochloric acid was added to adjust the pH to 3.5. In this manner, a virus inactivation treatment was carried out for one hour. This step was carried out at room temperature. After the virus inactivation treatment for one hour, the resultant solution was neutralized to pH 5.0 with a tris aqueous solution. The neutralized solution was loaded on a depth filter (Millistak (R) Pod A1HC) pre-washed with an acetate buffer (pH 5.0), and subsequently an acetate buffer (pH 5.0) was supplied for chasing to recover Mab A. Subsequently, filtration was carried out with a 0.2 $\mu$m filter and the filtrate was stored (depth filtration pool). The pH of the depth filtration pool was adjusted to 7.5 with a tris aqueous solution and used as an AEX material load. The AEX material load was passed through AEX Membrane Adsorber (NatriFlo (R) HD-Q) pre- equilibrated with a tris buffer (pH 7.5) containing 1 M sodium chloride, and subsequently with tris buffer (pH 7.5), and then, the AEX Membrane Adsorber was chased with tris buffer (pH 7.5) to recover a Mab A. After the pH of the recovered solution was adjusted to 5.0 with acetic acid, the solution filtered with a 0.2 $\mu$m filter. The filtrate was stored as an AEX material pool. The AEX material pool was passed through a CEX column packed with POROS XS, which was pre-equilibrated with an acetate buffer (pH 5.0) containing 1 M sodium chloride and subsequently with an acetate buffer (pH 5.0). The loading density on the CEX column was set to be 2000 g/L resin or less. After loading, an acetate buffer (pH 5.0) was supplied for chasing. The absorbance at 280 mm was monitored and fractions containing Mab A were obtained and used as CEX material pool by the F/T mode.

2) Results

The recovery rate of the Mab A in the F/T mode of the CEX chromatography was 92%. The results of clearance of impurities are shown in Table 2.

[Table 2]

| Table 2 Clearance of impurities in CEX chromatography (F/T mode) | | |
|---|---|---|
| Solution | Monomer content (%) | HCP content (ng/mg) |
| Cell culture supernatant | - | $2.1\times10^6$ |
| Affinity chromatography pool | 98.43 | $3.9\times10^3$ |
| Depth filtration pool | 97.93 | $7.5\times10^2$ |
| AEX material pool | 97.81 | $6.3\times10^2$ |
| CEX material pool (F/T mode) | 98.52 | $3.5\times10^1$ |

Example 3: Clearance of impurities by activated carbon material (Mab A, Mab B, Mab C, Mab D)

In this Example, clearance of impurities in a purification flow of a multiple antibodies referred to as Mab A, Mab B, Mab C and Mab D will be described in accordance with the purification flowchart shown in Table 3 below. Note that, the amino acid sequences of Mab A, Mab B, Mab C and Mab D, and antigens to be bound are mutually different.

[Table 3]

| Clarification of cell culture |
|---|
| ↓ |
| Affinity chromatography |
| ↓ |
| Virus inactivation |
| ↓ |
| Depth filtration |
| ↓ |
| Activated carbon filtration |

1) Clearance of impurities (except viruses) by activated carbon material

Millistak+ (R) Pod CR40 was used as the activated carbon material. Mab B and Mab A were evaluated for antibody recovery rate and clearance of impurities. The depth filtration pools of Mab B and Mab A obtained in accordance with the method shown in Example 2 were each used as a loading solution (activated carbon material load) to an activated carbon filter. Note that, in the case of Mab B, to facilitate evaluation of clearance performance of the activated carbon filter to remove HCP, washing a column with a phosphate buffer (pH 7.5) containing 1 M sodium chloride was not carried out in protein A chromatography after a cell culture supernatant was loaded. The activated carbon filter was appropriately pre-washed in accordance with an instruction manual and then equilibrated with an acetate buffer (pH 5.0). Note that the flow rate on and after equilibration step was set to be 109 LMH. After equilibration, the activated carbon material load in the case of Mab B was loaded at a volume of about 300 L/m$^2$; whereas, in the case of Mab A, the solution was loaded at a volume of about 117 L/m$^2$, and passed through. Thereafter, Mab B and Mab A were recovered with an acetate buffer (activated carbon material pool).

The purification conditions (Table 4), antibody recovery rates (Table 5), HCP clearances (Table 6), PLBL2 clearances (Table 7), and HMWS/LMWS clearances (Table 8) of individual antibodies are shown. In any one of the antibodies, the levels of HCP and PLBL2 were reduced by activated carbon filtration. Also it was confirmed that the contents of HMWS and LMWS were decreased and the content of a monomer was increased. In particular, the level of HCP was significantly reduced by activated carbon filtration (in Example 2, the removal rate of HCP before and after AEX chromatography was low and HCP loaded on CEX chromatography was not reduced). It was further found that the level of PLBL2 reported as a hitchhiker HCP, which is not easily reduced in accordance with a general antibody purification flow because it interacts with an antibody (International Publication No. WO2015/038888), can be reduced by activated carbon filtration. Since it is difficult to reduce the amount of PLBL2 (hitchhiker HCP) by a general purification method, reduction of about 50% of PLBL2 in load by an activated carbon material, in other words, obtaining a PLBL2 log reduction value (LRV) of 0.27 by an activated carbon material, has great significance.

[Table 4]

| Table 4 List of purification conditions of individual antibodies | | |
|---|---|---|
| Purification conditions | Mab B | Mab A |
| Washing with 1 M sodium chloride in affinity chromatography | not performed | performed |
| Volume loaded on activated carbon filter (L/m$^2$) | 300 | 117 |

[Table 5]

| Table 5 Antibody recovery rate (%) by activated carbon filtration | | |
|---|---|---|
| Solution | Mab B | Mab A |
| Activated carbon material pool | 95 | 94 |

[Table 6]

| Table 6 HCP clearance (ng/mg) by activated carbon filtration | | |
|---|---|---|
| Solution | Mab B | Mab A |
| Activated carbon material load | $2.2\times10^3$ | $6.1\times10^2$ |
| Activated carbon material pool | 6.0 | $6.0\times10^1$ |

[Table 7]

| Table 7 PLBL2 clearance (LRV) by activated carbon filtration | |
|---|---|
| Purification step | Mab A |
| Activated carbon filtration | 0.27 |

[Table 8]

| Table 8 HMWS/LMWS clearance (%) by activated carbon filtration | | |
|---|---|---|
| Solution | Mab B | Mab A |
| Activated carbon material load | HMWS:1.80 LMWS:0.94 Monomer:97.26 | HMWS:1.42 LMWS:0.24 Monomer:98.34 |
| Activated carbon material pool | HMWS:1.55 LMWS:0.51 Monomer:97.95 | HMWS:1.40 LMWS:0.19 Monomer: 98.41 |

2) Viral clearance by activated carbon material

Viral clearance was evaluated by using Millistak+ (R) Pod CR40 as an activated carbon material. Viral clearance performance by a spiking study was applied to Mab B, Mab C and Mab D. Viruses subjected to the spiking study were four types of viruses (MLV, MMV, Reo3 and PRV) generally used in viral clearance studies for therapeutic antibodies using CHO cells. As factors that may possibly have an effect on viral clearance performance by activated carbon filtration, flow rate, pH and type of antibody were used and studies examining the effects of these were designed. The depth filtration pools of Mab B, Mab C and Mab D obtained in accordance with the method shown in Example 2 were used as loading solutions (pH 5.0) to an activated carbon filter (activated carbon material load). A loading solution of pH 7.5 was prepared by adjusting the pH with a tris aqueous solution to 7.5. The activated carbon filter was appropriately pre-washed in accordance with an instruction manual, and thereafter, equilibrated with an acetate buffer (pH 5.0) or a tris buffer (pH 7.5). Note that the flow rate on and after equilibration step was set to be 109 LMH or 54.5 LMH. After equilibration, the activated carbon material was loaded at a volume of about 55 $L/m^2$ or about 110 $L/m^2$ and passed through. Thereafter, Mab B, Mab C and Mab D were recovered (activated carbon material pool) with an acetate buffer (pH 5.0) or a tris buffer (pH 7.5). A virus log reduction value (LRV) was calculated based on virus titers in the activated carbon material load and activated carbon material pool (Table 9). As a result, it was found that the activated carbon filter attains clearance of these four types of viruses. The viral clearance performance was not affected by the flow rate, the antibody types co-present and the load volume, and the LRV of the four types of viruses used in the viral clearance study for a therapeutic antibody using CHO cells were all high (i.e., > = 4). In contrast, in evaluating MLV, the viral clearance performance was compared between pH 5.0 and pH 7.5. As a result, it was found that the LRV was low at pH 7.5. From this, it was found that pH is an important parameter affecting viral clearance by an activated carbon material.

[Table 9]

| Table 9 Viral clearance (LRV) by activated carbon filtration | | | | | |
|---|---|---|---|---|---|
| Virus | pH | Flow rate (LMH) | Volume loaded ($L/m^2$) | Antibody | Virus log reduction value (LRV) |
| MLV | 5.0 | 109 | 110 | Mab C | >=5.26 |
| MLV | 5.0 | 54.5 | 110 | Mab C | >=5.09 |
| MLV | 7.5 | 109 | 110 | Mab C | 2.31 |
| MLV | 5.0 | 109 | 110 | Mab B | >=4.82 |
| MLV | 5.0 | 109 | 110 | Mab D | 4.81 |
| MMV | 5.0 | 109 | 55 | Mab C | 4.29 |
| MMV | 5.0 | 109 | 110 | Mab D | 5.87 |
| Reo3 | 5.0 | 109 | 55 | Mab C | >=7.67 |
| PRV | 5.0 | 109 | 55 | Mab C | 6.32 |

Example 4: Purification example by improved method 1 (Mab A, Mab C)

In this Example, clearance of impurities in a purification flow of monoclonal antibodies referred to as Mab A and Mab C will be described in accordance with the purification flowchart shown in Table 10 below.

[Table 10]

| Clarification of cell culture |
| :---: |
| ↓ |
| Affinity chromatography |
| ↓ |
| Virus inactivation |
| ↓ |
| Depth filtration |
| ↓ |
| Activated carbon filtration |
| ↓ |
| CEX chromatography (F/T mode) |

1) Materials and methods

The cell culture containing Mab A produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab A was eluted from the column with an acetate buffer (pH 3.6). The absorbance at 280 mm was monitored and fractions containing Mab A were obtained (affinity chromatography pool). To the affinity chromatography pool, hydrochloric acid was added to adjust the pH to 3.5. In this manner, a virus inactivation treatment was carried out for one hour. This step was carried out at room temperature. After the virus inactivation treatment for one hour, the resultant solution was neutralized to pH 5.0 with a tris aqueous solution and filtered with a 0.2 $\mu$m filter. The filtrate was stored (virus inactivated solution). The virus inactivated solution was loaded at a volume of about 109 L/m$^2$ on a depth filter (Millistak+ (R) Pod A1HC) pre-washed with an acetate buffer (pH 5.0), and subsequently, an acetate buffer (pH 5.0) was supplied for chasing. Mab A was recovered (depth filtration pool).

The depth filtration pool was loaded at a volume of 117 L/m$^2$ to an activated carbon filter (Millistak+ (R) Pod CR40) pre-washed with an acetate buffer (pH 5.0), and subsequently, an acetate buffer (pH 5.0) was supplied for chasing. Mab A was recovered. Subsequently, filtration was carried out with a 0.2 $\mu$m filter and the filtrate was stored (activated carbon material pool). The activated carbon material pool was passed through a CEX column packed with POROS XS pre-equilibrated with an acetate buffer (pH 5.0) containing 1 M sodium chloride, and subsequently with an acetate buffer (pH 5.0). The loading density in the CEX column was about 2000 g/L resin. After loading, an acetate buffer (pH 5.0) was supplied for chasing. The absorbance at 280 mm was monitored and fractions containing Mab A were obtained and used as a CEX material pool by the F/T mode.

Viral clearance was evaluated by using the affinity chromatography pool, the depth filtration pool and the activated carbon material pool of Mab C prepared in Example 3. Viruses subjected to the spiking study were four types of viruses (MLV, MMV, Reo3 and PRV) generally used in viral clearance studies of therapeutic antibodies using CHO cells. Based on virus titers, virus log reduction values (LRVs) were calculated.

2) Results

The results of Mab A recovery rate and clearance of impurities by improved method 1 are shown in Table 11 and Table 12, respectively. The total clearance in the purification steps achieving viral clearance in improved method 1, in the case of Mab C, is shown in Table 13.

The recovery rate of Mab A by improved method 1 was favorable. HMWS and LMWS were removed and an antibody having a high monomer content was obtained. The HCP amount was appropriately reduced. The PLBL2 amount was successfully reduced. It was also confirmed that DNA was removed to less than the quantification limit.

The total viral clearance in the purification steps having viral clearance performance in improved method 1, in the case of Mab C, was a favorable result.

It was found that purification achieving high purity can be done by using a treatment with an activated carbon material in combination; and that viruses can be removed by the treatment with an activated carbon material. Based on the findings, in the method shown in Example 2, replacement of the AEX chromatography with an activated carbon material became possible. As to the production cost, since the loading density of about 100 g/L resin can be increased to about 2000 g/L resin by replacing the B/E mode of CEX chromatography generally used with the F/T mode, the required cost for an expensive CEX resin can be reduced to about 1/20. In addition, since expensive AEX resin and membrane can be replaced with an inexpensive activated carbon material, a reduction of production cost was attained. Furthermore, when AEX chromatography was taken away and a filter cartridge-type depth filter, followed by a filter cartridge-type activated carbon filter was used, the production period can be reduced by one day. Since it is necessary for many pharmaceutical manufacturers to attain efficiency in production, which is realized by maximizing the number of production lots per year, a one-day reduction of the production period has great significance.

Due to the improved method, the purity was increased to a sufficient level for therapeutic use in humans; the production cost and production period were reduced, with the result that an antibody was able to be purified efficiently.

[Table 11]

| Table 11 Recovery rate by improved method 1 (only improved steps are listed) | |
|---|---|
| Purification step | Recovery rate (%) |
| Depth filtration | 96 |
| Activated carbon filtration | 94 |
| CEX chromatography (F/T mode) | 94 |

[Table 12]

| Table 12 Clearance of impurities by improved method 1 | | | | |
|---|---|---|---|---|
| Solution | Monomer content (%) | HCP content (ng/mg) | PLBL2 content (LRV) | DNA content (pg/mg) |
| Cell culture supernatant | N.T. | N.T. | - | $1.52 \times 10^2$ |
| Affinity chromatography pool | N.T. | N.T. | 0.42 | 5.50 |
| Virus inactivated solution | 97.99 | $2.12 \times 10^3$ | 0.13 | 2.40 |
| Depth filtration pool | 98.34 | $6.12 \times 10^2$ | 0.15 | <QL |
| Activated carbon material pool | 98.45 | $4.70 \times 10^1$ | 0.28 | <QL |
| CEX material pool (F/T mode) | 98.77 | $1.30 \times 10^1$ | 0.06 | <QL |
| (QL: quantification limit) | | | | |

[Table 13]

| Table 13 Viral clearance (LRV) by improved method 1 | | | | |
|---|---|---|---|---|
| Purification step | MLV | MMV | Reo3 | PRV |
| Virus inactivation | 5.71 | N.T. | N.T. | N.T. |
| Activated carbon filtration | >=5.26 | 4.29 | >=7.67 | 6.32 |
| CEX chromatography (F/T mode) | 6.09 | 2.62 | 8.03 | >=5.71 |
| Total clearance in purification step | >=17.06 | >=6.91 | >=15.70 | >=12.03 |

Example 5: Purification example by improved method 2 (Mab A, Mab D)

In this Example, clearance of impurities in a purification flow of monoclonal antibodies referred to as Mab A and Mab D in accordance with the purification flowchart shown in Table 14 below, in which depth filtration was omitted from the purification flowchart of Example 4 and shown in Table 10 will be described. Note that, the amino acid sequence of Mab D and antigen to be bound are different from those of Mab A, Mab B and Mab C.

[Table 14]

| Clarification of cell culture |
| :---: |
| ↓ |
| Affinity chromatography |
| ↓ |
| Virus inactivation |
| ↓ |
| Activated carbon filtration |
| ↓ |
| CEX chromatography (F/T mode) |

    1) Materials and methods
    1)-1) Case of Mab A

The cell culture containing Mab A produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab A was eluted from the column with an acetate buffer (pH 3.6). The absorbance at 280 mm was monitored and fractions containing each of the antibodies were obtained (affinity chromatography pool). To the affinity chromatography pool, hydrochloric acid was added to adjust the pH to 3.5. In this manner, a virus inactivation treatment was carried out for one hour. This step was carried out at room temperature. After the virus inactivation treatment for one hour, the resultant solution was neutralized to pH 5.0 with a tris aqueous solution and filtered with a 0.2 $\mu$m filter. The filtrate was stored (virus inactivated solution). The virus inactivated solution was loaded at a volume of 70 L/m$^2$ to an activated carbon filter (Millistak+ (R) Pod CR40) pre-washed with an acetate buffer (pH 5.0), and subsequently an acetate buffer (pH 5.0) was supplied for chasing. The antibodies were recovered. Subsequently, filtration was carried out with a 0.2 $\mu$m filter and the filtrate was stored (activated carbon material pool). The activated carbon material pool was passed through a CEX column packed with POROS XS pre-equilibrated with an acetate buffer (pH 5.0) containing 1 M sodium chloride, and subsequently with an acetate buffer (pH 5.0). The loading density in the CEX column was set to be about 2000 g/L resin. After loading, an acetate buffer (pH 5.0) was supplied for chasing. The absorbance at 280 mm was monitored and fractions containing each of the antibodies were obtained and subsequently filtered with a 0.2 $\mu$m filter to obtain a CEX material pool by the F/T mode.

1)-2) Case of Mab D The cell culture containing Mab D produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab D was eluted from the column with an acetate buffer (pH 3.6). The absorbance at 280 mm was monitored and fractions containing each antibody were obtained (affinity chromatography pool). To the affinity chromatography pool, hydrochloric acid was added to adjust the pH to 3.4. In this manner, a virus inactivation treatment was carried out for one hour. This step was carried out at room temperature. After the virus inactivation treatment for one hour, the resultant solution was neutralized to pH 5.0 with a tris aqueous solution and stored (virus inactivated solution). The virus inactivated solution was loaded at a volume of 178 L/m$^2$ to an activated carbon filter (Millistak+ (R) Pod CR40) pre-washed with an acetate buffer (pH 5.0), and subsequently, an acetate buffer (pH 5.0) was supplied for chasing. The antibodies were recovered. Subsequently, filtration with a 0.2 μm filter was carried out and the filtrate was stored (activated carbon material pool).

The activated carbon material pool was passed through a CEX column packed with POROS XS pre-equilibrated with an acetate buffer (pH 5.0) containing 1 M sodium chloride, and subsequently with an acetate buffer (pH 5.0). The loading density in the CEX column was set to be about 1500 g/L resin. After loading, an acetate buffer (pH 5.0) was supplied for chasing. The absorbance at 280 mm was monitored. Fractions containing each of the antibodies were obtained and subsequently filtered with a 0.2 μm filter to obtain a CEX material pool by the F/T mode.

2) Results

The results of Mab A recovery rate and clearance of impurities by improved method 2 are shown in Table 15 and Table 16, respectively; and the results of Mab D recovery rate and clearance of impurities are shown in Table 17 and Table 18, respectively. Note that, the total viral clearance in the purification steps having viral clearance performance in improved method 2 can be expected to be the same as that shown in Table 13 in Example 4 because the steps are the same.

The recovery rates of Mab A and Mab D by improved method 2 were both favorable. HMWS and LMWS were removed and antibodies having a high monomer content were obtained. In the case of Mab D, the HCP was sufficiently removed. It was confirmed that DNA was removed to a lower level than the quantification limit. In addition, the contents of PLBL2 and leached protein A were successfully reduced. From the results, it was demonstrated that some antibodies can be purified to a high degree of purity by the purification method even if depth filtration is not carried out.

[Table 15]

| Table 15 Recovery rate by improved method 2 (only improved steps are listed, Mab A) | |
|---|---|
| Purification step | Recovery rate (%) |
| Activated carbon filtration | 93 |
| CEX chromatography (F/T mode) | 85 |

[Table 16]

| Table 16 Clearance of impurities by improved method 2 (Mab A) | | | | | |
|---|---|---|---|---|---|
| Solution | Monomer content (%) | HCP content (ng/mq) | PLBL2 content (LRV) | DNA content (pg/mg) | Content (ng/mg) of exudate protein A (ng/mg) |
| Cell culture supernatant | N.T. | N.T. | - | $1.52\times10^2$ | N.T. |
| Affinity chromatography pool | N.T. | N.T. | 0.42 | 5.50 | $5.86\times10^1$ |
| Virus inactivated solution | 98.13 | $2.06\times10^3$ | 0.13 | 2.40 | $5.98\times10^1$ |
| Activated carbon material pool | 98.31 | $6.05\times10^2$ | 0.30 | <QL | $2.32\times10^1$ |
| CEX material pool (F/T mode) | 98.79 | $4.00\times10^1$ | 0.14 | <QL | $1.55\times10^1$ |

[Table 17]

| Table 17 Recovery rate by improved method 2 (only improved steps are listed, Mab D) | |
|---|---|
| Purification step | Recovery rate (%) |
| Activated carbon filtration | 100 |
| CEX chromatography (F/T mode) | 90 |

[Table 18]

| Table 18 Clearance of impurities by improved method 2 (Mab D) | | | | | |
|---|---|---|---|---|---|
| Solution | Monomer content (%) | HCP content (ng/mg) | PLBL2 content (LRV) | DNA content (pg/mg) | Content (ng/mg) of exudate protein A(ng/mg) |
| Cell culture supernatant | N.T. | $7.13 \times 10^4$ | - | $3.1 \times 10^3$ | N.T. |
| Affinity chromatography pool | 98.49 | $8.70 \times 10^1$ | 0.94 | < QL | $1.40 \times 10^1$ |
| Virus inactivated solution | 97.65 | $3.90 \times 10^1$ | 0.01 | < QL | $1.10 \times 10^1$ |
| Activated carbon material pool | 97.96 | $1.70 \times 10^1$ | 0.59 | < QL | 7.40 |
| CEX material pool (F/T mode) | 99.02 | $3.00 \times 10^0$ | -0.13 | < QL | 4.25 |

Example 6: Purification example by improved method 3 (Mab F, Mab G)

In this Example, clearance of impurities in a purification flow of antibodies will be described in accordance with the purification flowchart shown in Table 19 below, in which a hydrophobic interaction chromatography was added after the cation exchange chromatography in the purification flowchart shown in Table 10 in Example 4. Note that, the amino acid sequences of Mab E, Mab F and Mab G used in this Example and antigens to be bound are mutually different and also different from those of Mab A, Mab B, Mab C and Mab D.

[Table 19]

| Clarification of cell culture |
|---|
| ↓ |
| Affinity chromatography |
| ↓ |
| Virus inactivation |
| ↓ |
| Depth filtration |
| ↓ |
| Activated carbon filtration |
| ↓ |
| CEX chromatography (F/T mode) |
| ↓ |
| Hydrophobic interaction chromatography |

1) Viral clearance by hydrophobic interaction chromatography (Mab E)

In this Example, viral clearance in a purification flow of monoclonal antibody referred to as Mab E by hydrophobic interaction chromatography will be described. Viral clearance was evaluated by a spiking study of MLV and MMV to Mab E using POROS Benzyl Ultra HIC resin.

The cell culture containing Mab E produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab E was eluted from the column with an acetate buffer (pH 3.6). The absorbance at 280 mm was monitored and fractions containing Mab E were obtained (affinity chromatography pool). To the affinity chromatography pool, hydrochloric acid was added to adjust the pH to 3.4. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at a volume of 114 $L/m^2$ on a depth filter (Millistak+ (R) Pod A1HC) pre-washed with an acetate buffer (pH 5.0), and subsequently an acetate buffer (pH 5.0) was supplied for chasing. Mab E was recovered (depth filtration pool). The depth filtration pool was loaded at a volume of 129 $L/m^2$ on an activated carbon filter (Millistak+ (R) Pod CR40) pre-washed with an acetate buffer (pH 5.0), and subsequently an acetate buffer (pH 5.0) was supplied for chasing. Filtration was carried out with a 0.2 $\mu$m filter and the filtrate was stored (activated carbon material pool). The activated carbon material pool was passed through an HIC column packed with POROS Benzyl Ultra and pre-equilibrated with water for injection and subsequently with an acetate buffer (pH 5.0). The loading density on the HIC column was about 1000 g/L resin. After loading, an acetate buffer (pH 5.0) was supplied for chasing. The absorbance at 280 mm was monitored and fractions containing Mab E were obtained and used as an HIC material pool by the F/T mode. Based on virus titers in the loading solution and pool solution, virus log reduction values (LRVs) were calculated (Table 20). As a result, it was found that the hydrophobic interaction chromatography attained viral clearance.

[Table 20]

| Table 20 Viral clearance LRV) by hydrophobic interaction chromatography | |
|---|---|
| Virus | Virus log reduction value (LRV) |
| MLV | 2.92 |
| MMV | 4.22 |

2) Clearance of impurities by improved method 3 (Mab F)

In this Example, clearance of impurities in a purification flow of a monoclonal antibody referred to as Mab F will be described in accordance with the purification flowchart shown in Table 19.

The cell culture containing Mab F produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab F was eluted from the column with an acetate buffer (pH 3.6). The absorbance at 280 mm was monitored and fractions containing Mab F were obtained (affinity chromatography pool). To the affinity chromatography pool, hydrochloric acid was added to adjust the pH to 3.7. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at an amount of about 900 $g/m^2$ on a depth filter (Millistak+ (R) Pod A1HC) pre-washed with an acetate buffer (pH 5.0), and subsequently, an acetate buffer (pH 5.0) was supplied for chasing. Mab F was recovered (depth filtration pool). The depth filtration pool was loaded at an amount of about 800 $g/m^2$ on an activated carbon filter (Millistak+ (R) Pod CR40) pre-washed with an acetate buffer (pH 5.0), and subsequently an acetate buffer (pH 5.0) was supplied for chasing. Filtration was carried out with a 0.2 $\mu$m filter and the filtrate was stored (activated carbon material pool). The activated carbon material pool was passed through a CEX column packed with POROS XS and pre-equilibrated with an acetate buffer (pH 5.0) containing 1 M sodium chloride and subsequently with an acetate buffer (pH 5.0). The loading density on the CEX column was set to be about 1300 g/L resin. After loading, an acetate buffer (pH 5.0) was supplied for chasing. The absorbance at 280 mm was monitored and fractions containing Mab F were obtained and subsequently filtered with a 0.2 μm filter. The filtrate was used as a CEX material pool by the F/T mode. The CEX material pool was passed through an HIC column packed with POROS Benzyl Ultra and pre-equilibrated with water for injection and subsequently with an acetate buffer (pH 5.0). The loading density on the HIC column was about 300 g/L resin. After loading, an acetate buffer (pH 5.0) was supplied for chasing. The absorbance at 280 mm was monitored and fractions containing Mab F were obtained and used as an HIC material pool by the F/T mode.

The results of clearance of impurities by improved method 3 are shown in Table 21. The monomer was increased by the hydrophobic interaction chromatography. Residual HCP and PLBL2 after cation exchange chromatography were removed by the hydrophobic interaction chromatography; in particular, PLBL2 was removed to less than the quantification limit.

[Table 21]

| Table 21 Clearance of impurities by improved method 3 (Mab F) | | | |
|---|---|---|---|
| Solution | Monomer content (%) | HCP content (ng/mg) | PLBL2 content (ng/mg) |
| Cell culture supernatant | N.T. | $2.87 \times 10^5$ | $1.11 \times 10^3$ |
| Affinity chromatography pool | 98.17 | $2.74 \times 10^2$ | $4.21 \times 10^2$ |
| Virus inactivated solution | 96.60 | N.T. | N.T. |
| Depth filtration pool | 97.15 | $8.61 \times 10^1$ | $1.71 \times 10^2$ |
| Activated carbon material pool | 97.77 | $7.75 \times 10^1$ | $1.14 \times 10^2$ |
| CEX material pool (F/T mode) | 98.30 | $2.12 \times 10^1$ | $9.74 \times 10^1$ |
| HIC material pool (F/T mode) | 98.55 | $1.14 \times 10^1$ | < 0.35 |

3) Clearance of impurities by improved method 3 (Mab G)

In this Example, clearance of impurities in a purification flow of a monoclonal antibody referred to as Mab G will be described in accordance with the purification flowchart shown in Table 19.

The cell culture containing Mab G produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab G was eluted from the column with an acetate buffer (pH 3.6). The absorbance at 280 mm was monitored and fractions containing Mab G were obtained (affinity chromatography pool). To the affinity chromatography pool, hydrochloric acid was added to adjust the pH to 3.7. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at an amount of about 800 g/m² on a depth filter (Millistak+ (R) Pod A1HC) pre-washed with an acetate buffer (pH 5.0) and subsequently an acetate buffer (pH 5.0) was supplied for chasing. Mab G was recovered (depth filtration pool). The depth filtration pool was loaded at an amount of about 700 g/m² on an activated carbon filter (Millistak+ (R) Pod CR40) pre-washed with an acetate buffer (pH 5.0), and subsequently an acetate buffer (pH 5.0) was supplied for chasing. Filtration was carried out with a 0.2 μm filter and the filtrate was stored (activated carbon material pool). The activated carbon material pool was passed through a CEX column packed with POROS XS and pre-equilibrated with an acetate buffer (pH 5.0) containing 1 M sodium chloride and subsequently with an acetate buffer (pH 5.0). The loading density on the CEX column was set to be about 1500 g/L resin. After loading, an acetate buffer (pH 5.0) was supplied for chasing. The absorbance at 280 mm was monitored and fractions containing Mab G were obtained and subsequently filtered with a 0.2 μm filter to obtain a CEX material pool by the F/T mode. The CEX material pool was passed through an HIC column packed with POROS Benzyl Ultra and pre-equilibrated with water for injection and subsequently with an acetate buffer (pH 5.0). The loading density on the HIC column was about 300 g/L resin. After loading, an acetate buffer (pH 5.0) was supplied for chasing. The absorbance at 280 mm was monitored and fractions containing Mab G were obtained and used as an HIC material pool by the F/T mode.

The results of clearance of impurities by improved method 3 are shown in Table 22. The monomer was increased by the hydrophobic interaction chromatography. Residual HCP and PLBL2 after cation exchange chromatography were removed by the hydrophobic interaction chromatography; in particular, PLBL2 was removed to around the quantification limit.

[Table 22]

| Table 22 Clearance of impurities by improved method 3 (Mab G) | | | |
|---|---|---|---|
| Solution | Monomer content (%) | HCP content (ng/mg) | PLBL2 content (ng/mg) |
| Cell culture supernatant | N.T. | $3.51 \times 10^5$ | $1.39 \times 10^3$ |
| Affinity chromatography pool | 97.88 | $8.88 \times 10^2$ | $3.92 \times 10^2$ |
| Virus inactivated solution | 96.90 | N.T. | N.T. |
| Depth filtration pool | 97.44 | $1.52 \times 10^2$ | $2.18 \times 10^2$ |
| Activated carbon material pool | 97.94 | $9.76 \times 10^1$ | $1.53 \times 10^2$ |
| CEX material pool (F/T mode) | 98.32 | $1.84 \times 10^1$ | $1.52 \times 10^2$ |
| HIC material pool (F/T mode) | 98.63 | $8.14 \times 10^0$ | 0.44 |

Example: 7 Purification example by improved method 4 (Mab F, Mab G)

In this Example, clearance of impurities in a purification flow of antibodies will be described in accordance with the purification flowchart shown in Table 23 below, in which a hydrophobic interaction chromatography was added before the cation exchange chromatography in the purification flowchart shown in Table 10 in Example 4.

[Table 23]

| Clarification of cell culture |
|---|
| ↓ |
| Affinity chromatography |
| ↓ |
| Virus inactivation |
| ↓ |
| Depth filtration |
| ↓ |
| Activated carbon filtration |
| ↓ |
| Hydrophobic interaction chromatography |
| ↓ |
| CEX chromatography (F/T mode) |

1) Clearance of impurities by improved method 4 (Mab F)

In this Example, clearance of impurities in a purification flow of a monoclonal antibody referred to as Mab F will be described in accordance with the purification flowchart shown in Table 23.

The cell culture containing Mab F produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab F was eluted from the column with an acetate buffer (pH 3.6). The absorbance at 280 mm was monitored and fractions containing Mab F were obtained (affinity chromatography pool). To the affinity chromatography pool, hydrochloric acid was added to adjust the pH to 3.7. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at an amount of about 900 g/m$^2$ on a depth filter (Millistak+ (R) Pod A1HC) pre-washed with an acetate buffer (pH 5.0), and subsequently an acetate buffer (pH 5.0) was supplied for chasing. Mab F was recovered (depth filtration pool). The depth filtration pool was loaded at an amount of about 800 g/m$^2$ on an activated carbon filter (Millistak+ (R) Pod CR40) pre-washed with an acetate buffer (pH 5.0), and subsequently an acetate buffer (pH 5.0) was supplied for chasing. Filtration was carried out with a 0.2 $\mu$m filter and the filtrate was stored (activated carbon material pool). The activated carbon material pool was passed through an HIC column packed with POROS Benzyl Ultra and pre-equilibrated with water for injection and subsequently with an acetate buffer (pH 5.0). The loading density on the HIC column was about 300 g/L resin. After loading, an acetate buffer (pH 5.0) was supplied for chasing. The absorbance at 280 mm was monitored and fractions containing Mab F were obtained and used as an HIC material pool by the F/T mode. The HIC material pool was passed through a CEX column packed with POROS XS and pre-equilibrated with an acetate buffer (pH 5.0) containing 1 M sodium chloride, and subsequently with an acetate buffer (pH 5.0). The loading density on the CEX column was set to be about 1300 g/L resin. After loading, an acetate buffer (pH 5.0) was supplied for chasing. The absorbance at 280 mm was monitored and fractions containing Mab F were obtained and subsequently filtered with a 0.2 $\mu$m filter to obtain a CEX material pool by the F/T mode.

The results of clearance of impurities by improved method 4 are shown in Table 24. The monomer was increased by the hydrophobic interaction chromatography. Residual HCP and PLBL2 in the activated carbon material pool were removed by the hydrophobic interaction chromatography; in particular, PLBL2 was removed to around the quantification limit.

[Table 24]

| Table 24 Clearance of impurities by improved method 4 (Mab F) | | | |
|---|---|---|---|
| Solution | Monomer content (%) | HCP content (ng/mg) | PLBL2 content (ng/mg) |
| Cell culture supernatant | N.T. | $2.87 \times 10^5$ | $1.11 \times 10^3$ |
| Affinity chromatography pool | 98.17 | $2.74 \times 10^2$ | $4.21 \times 10^2$ |
| Virus inactivated solution | 96.60 | N.T. | N.T. |
| Depth filtration pool | 97.15 | $8.61 \times 10^1$ | $1.71 \times 10^2$ |
| Activated carbon material pool | 97.77 | $7.75 \times 10^1$ | $1.14 \times 10^2$ |
| HIC material pool (F/T mode) | 98.26 | $4.31 \times 10^1$ | $6.50 \times 10^{-1}$ |
| CEX material pool (F/T mode) | 98.60 | < 10.52 | $6.60 \times 10^{-1}$ |

2) Clearance of impurities by improved method 4 (Mab G)

In this Example, clearance of impurities in a purification flow of a monoclonal antibody referred to as Mab G will be described in accordance with the purification flowchart shown in Table 23.

The cell culture containing Mab G produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab G was eluted from the column with an acetate buffer (pH 3.6). The absorbance at 280 mm was monitored and fractions containing Mab G were obtained (affinity chromatography pool). To the affinity chromatography pool, hydrochloric acid was added to adjust the pH to 3.7. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at an amount of about 800 g/m$^2$ on a depth filter (Millistak+ (R) Pod A1HC) pre-washed with an acetate buffer (pH 5.0), and subsequently, an acetate buffer (pH 5.0) was supplied for chasing. Mab G was recovered (depth filtration pool). The depth filtration pool was loaded at an amount of about 700 g/m$^2$ on an activated carbon filter (Millistak+ (R) Pod CR40) pre-washed with an acetate buffer (pH 5.0), and subsequently an acetate buffer (pH 5.0) was supplied for chasing. Filtration was carried out with a 0.2 $\mu$m filter and the filtrate was stored (activated carbon material pool). The activated carbon material pool was passed through an HIC column packed with POROS Benzyl Ultra and pre-equilibrated with water for injection, and subsequently with an acetate buffer (pH 5.0). The loading density on the HIC column was about 300 g/L resin. After loading, an acetate buffer (pH 5.0) was supplied for chasing. The absorbance at 280 mm was monitored and fractions containing Mab G were obtained and used as an HIC material pool by the F/T mode. The HIC material pool was passed through a CEX column packed with POROS XS pre-equilibrated with an acetate buffer (pH 5.0) containing 1 M sodium chloride, and subsequently with an acetate buffer (pH 5.0). The loading density on the CEX column was set to be about 1300 g/L resin. After loading, an acetate buffer (pH 5.0) was supplied for chasing. The absorbance at 280 mm was monitored and fractions containing Mab G were obtained and subsequently filtered with a 0.2 $\mu$m filter to obtain a CEX material pool by the F/T mode.

The results of clearance of impurities by improved method 4 are shown in Table 25. The monomer was increased by the hydrophobic interaction chromatography. Residual HCP and PLBL2 in the activated carbon material pool were removed by the hydrophobic interaction chromatography; in particular, PLBL2 was removed to around the quantification limit.

[Table 25]

| Table 25 Clearance of impurities by improved method 4 (Mab G) | | | |
|---|---|---|---|
| Solution | Monomer content (%) | HCP content (ng/mg) | PLBL2 content (ng/mg) |
| Cell culture supernatant | N.T. | $3.51 \times 10^5$ | $1.39 \times 10^3$ |
| Affinity chromatography pool | 97.88 | $8.88 \times 10^2$ | $3.92 \times 10^2$ |
| Virus inactivated solution | 96.90 | N.T. | N.T. |
| Depth filtration pool | 97.44 | $1.52 \times 10^2$ | $2.18 \times 10^2$ |
| Activated carbon material pool | 97.94 | $9.76 \times 10^1$ | $1.53 \times 10^2$ |
| HIC material pool (F/T mode) | 98.31 | $7.05 \times 10^1$ | $1.11 \times 10^0$ |
| CEX material pool (F/T mode) | 98.62 | $5.05 \times 10^0$ | $1.03 \times 10^0$ |

Example 8: Clearance of impurities by an activated carbon material (Mab A)

In this Example, clearance of impurities in a purification flow of a monoclonal antibody referred to as Mab A by an activated carbon material will be described in accordance with the purification flowchart shown in Table 26 below.

[Table 26]

| Clarification of cell culture |
|---|
| ↓ |
| Affinity chromatography |
| ↓ |
| Virus inactivation |
| ↓ |
| Depth filtration |
| ↓ |
| Activated carbon filtration |

1) Materials and methods

The cell culture containing Mab A produced by CHO cells was filtered with a depth filter to remove the cells and subjected to clarification. The cell culture supernatant was purified by protein A chromatography using KanCapA resin. After the column was equilibrated with a phosphate buffer (pH 7.5) containing 20 mM sodium chloride, the cell culture supernatant was loaded. Thereafter, the column was washed with a phosphate buffer (pH 7.5) containing 1 M sodium chloride and subsequently with a phosphate buffer (pH 7.5) containing 20 mM sodium chloride. Mab A was eluted from the column with an acetate buffer (pH 3.6). The absorbance at 280 mm was monitored and fractions containing Mab A were obtained (affinity chromatography pool). To the affinity chromatography pool, hydrochloric acid was added to adjust the pH to 3.5. In this manner, a virus inactivation treatment was carried out for one hour. This step was carried out at room temperature. After the virus inactivation treatment for one hour, the resultant solution was neutralized to pH 5.0 with a tris aqueous solution (virus inactivated solution). The virus inactivated solution was loaded at a volume of 124 L/m$^2$ on a depth filter (Millistak+ (R) Pod A1HC) pre-washed with an acetate buffer (pH 5.0), and subsequently an acetate buffer (pH 5.0) was supplied for chasing. Mab A was recovered (depth filtration pool). The depth filtration pool was adjusted by hydrochloric acid or a tris aqueous solution so as to have pH 4.5, pH 5.0, pH 5.5, pH 6.0, pH 6.5, pH 7.0 or pH 7.5 and filtered with a 0.2 $\mu$m filter. The filtrate was stored (pH-adjusted activated carbon material load). The depth filtration pool was adjusted by ultrafiltration/diafiltration so as to have an electrical conductivity of 2 mS/cm, 4 mS/cm, 5 mS/cm, 6 mS/cm, 8 mS/cm or 10 mS/cm and filtered with a 0.2 $\mu$m filter. The filtrate was stored (electrical conductivity-adjusted activated carbon material load).

The pH-adjusted activated carbon material load was loaded at a volume of 60 L/m$^2$ or 100 L/m$^2$ to activated carbon filter (Millistak+ (R) Pod CR40) pre-washed with an acetate buffer (pH 4.5, 5.0, 5.5, 6.0, 6.5, pH 7.0, pH 7.5) having the same pH as the loading solution, and subsequently an acetate buffer (pH 4.5, pH 5.0, pH 5.5, pH 6.0, pH 6.5, pH 7.0, pH 7.5) having the same pH as the loading solution was supplied for chasing. Mab A was recovered as a pH-adjusted activated carbon material pool. The electrical conductivity-adjusted activated carbon material load was loaded at a volume of 60 L/m$^2$ to an activated carbon filter (Millistak+ (R) Pod CR40) washed with an acetate buffer (pH 5.0), and subsequently an acetate buffer (pH 5.0) was supplied for chasing. Mab A was recovered as an electrical conductivity-adjusted activated carbon material pool.

2) Results

The results of clearance of impurities by the activated carbon filtration (load volume: 60 L/m$^2$) in the purification flowchart shown in Table 26, are shown in Table 27 and Table 28. The HCP removal rate was not significantly affected by pH and electrical conductivity. There was a tendency that the removal rate of PLBL2 increases as the pH decreases or as the electrical conductivity decreases. There was a tendency that the removal rate of leached protein A increases as the pH decreases. There was a tendency that the removal rates of MLV and MMV increase as the pH decreases or the electrical conductivity decreases. It was confirmed that the conditions of pH and electrical conductivity of the activated carbon material load obtained in accordance with the purification flowchart shown in Table 26 are those providing high impurity removal rates by the activated carbon filtration.

[Table 27]

| Table 27 pH PH and clearance of impurities (LRV) by activated carbon filtration | | | | | |
|------|------|-------|------------------|-------|------|
| pH | HCP | PLBL2 | Leached protein A | MLV | MMV |
| 4.5 | 0.16 | 0.42 | 0.31 | >2.43 | 1.69 |
| 5.0 | 0.25 | 0.27 | 0.24 | 2.74 | 2.31 |
| 5.5 | 0.16 | 0.09 | 0.12 | 1.19 | 1.94 |
| 6.0 | 0.16 | 0.03 | 0.16 | 1.38 | 1.19 |
| 6.5 | 0.17 | 0.07 | 0.05 | 1.56 | 0.56 |
| 7.0 | 0.17 | 0.07 | 0.05 | 1.00 | 0.19 |
| 7.5 | 0.17 | 0.00 | 0.01 | 1.06 | 0.25 |

[Table 28]

| Table 28 Electrical conductivity and clearance of impurities (LRV) by activated carbon filtratio | | | | | |
|------------------------|------|-------|-------------------|------|------|
| Electrical conductivity | HCP | PLBL2 | Leached protein A | MLV | MMV |
| 2 mS/cm | 0.20 | 0.93 | >0.20 | 4.92 | 4.64 |
| 4 mS/cm | 0.22 | 0.63 | >0.09 | 5.24 | 5.14 |
| 5 mS/cm | 0.18 | 0.51 | >0.11 | 4.14 | 5.20 |
| 6 mS/cm | 0.21 | 0.34 | >0.16 | 3.77 | 4.36 |
| 8 mS/cm | 0.16 | 0.24 | >0.08 | 3.03 | 4.20 |
| 10 mS/cm | 0.19 | 0.13 | >-0.06 | 3.04 | 4.22 |

Industrial Applicability

Due to the antibody purification method of the present invention, it becomes possible to reduce the purification period, remove impurities efficiently, purify an antibody to a sufficient degree of purity for therapeutic use in humans, and reduce production cost.

**Claims**

1.  A method for purifying an antibody from a composition containing one or more impurities, comprising the steps of:

    a1. providing a cell culture supernatant containing the antibody;
    b1. processing the cell culture supernatant through an affinity chromatography resin to obtain an affinity chromatography pool containing the antibody;
    c1. inactivating viruses in the affinity chromatography pool to obtain a virus inactivated solution;
    d1. treating the virus inactivated solution with an activated carbon material to obtain an activated carbon material pool; and
    e1. processing the activated carbon material pool through a cation exchange material to obtain a cation exchange material pool.

2.  The method for purifying an antibody from a composition containing one or more impurities according to claim 1, comprising the steps of:

    a2. providing a cell culture supernatant containing the antibody;
    b2. processing the cell culture supernatant through an affinity chromatography resin to obtain an affinity chromatography pool containing the antibody;
    c2. inactivating viruses in the affinity chromatography pool to obtain a virus inactivated solution;
    d2. processing the virus inactivated solution through a depth filter to obtain a depth filtration pool;
    e2. treating the depth filtration pool with an activated carbon material to obtain an activated carbon material pool; and
    f2. processing the activated carbon material pool through a cation exchange material to obtain a cation exchange material pool.

3.  The method according to claim 1 or 2, wherein the affinity chromatography resin is a protein A resin.

4.  The method according to claim 3, wherein the protein A resin is KanCapA, KanCapA3G, MabSelect SuRe, MabSelect SuRe pcc, MabSelect PrismA or Amsphere A3.

5.  The method according to any one of claims 1 to 4, wherein the virus inactivation is a treatment with at least one selected from the group consisting of an acid, a surfactant, a chemical substance, a nucleic acid crosslinking agent, ultraviolet rays, gamma rays and heat.

6.  The method according to claim 5, wherein the virus inactivation comprises lowering pH of the affinity chromatography pool to 3 to 4.

7.  The method according to claim 6, wherein the affinity chromatography pool is incubated for 60 to 120 minutes during the virus inactivation.

8.  The method according to claim 6 or 7, wherein the affinity chromatography pool is incubated at 15 to 30°C during the virus inactivation.

9.  The method according to any one of claims 1 to 8, wherein the solution to be loaded on the activated carbon material, and an equilibration buffer and a wash buffer for the activated carbon material each have a pH of 4.5 to 7.5.

10. The method according to claim 9, wherein the solution to be loaded on the activated carbon material, and the equilibration buffer and the wash buffer for the activated carbon material each have a pH of 4.5 to 5.5.

11. The method according to any one of claims 1 to 10, wherein the solution to be loaded on the activated carbon material, and an equilibration buffer and a wash buffer for the activated carbon material each have an electrical conductivity of 2 to 10 mS/cm.

12. The method according to claim 11, wherein the solution to be loaded on the activated carbon material, and the equilibration buffer and the wash buffer for the activated carbon material each have an electrical conductivity of 2 to 5 mS/cm.

13. The method according to any one of claims 1 to 12, wherein the treatment with an activated carbon material is carried out by use of a filter containing the activated carbon material.

14. The method according to claim 13, wherein the filter is Millistak+ Pod CR40, Zeta Plus activated carbon adsorption depth filter or SUPRAcap 50 Seitz AKS filter.

15. The method according to any one of claims 1 to 14, wherein the activated carbon material pool is loaded on the cation exchange material in an amount exceeding the binding capacity of the cation exchange material.

16. The method according to claim 15, wherein the activated carbon material pool is loaded on the cation exchange material at a loading density of 500 to 2000 g/L.

17. The method according to claim 16, wherein the activated carbon material pool is loaded on the cation exchange material at a loading density of 1000 to 1500 g/L.

18. The method according to any one of claims 1 to 17, wherein the cation exchange material is a resin, a monolith column or a membrane.

19. The method according to claim 18, wherein the cation exchange material has a functional group, which is sulfopropyl, sulfoethyl, sulfoisobutyl or carboxyl.

20. The method according to claim 19, wherein the cation exchange material is POROS XS, Eshmuno CPX, Eshmuno CP-FT or CaptoS ImpAct.

21. The method according to any one of claims 1 to 20, wherein the solution to be loaded on the cation exchange material has a pH of 4 to 6.

22. The method according to claim 21, wherein the solution to be loaded on the cation exchange material has a pH of 4.5 to 5.5.

23.  The method according to any one of claims 1 to 22, wherein the solution to be loaded on the cation exchange material has an electrical conductivity of 3 to 7 mS/cm.

24. The method according to claim 23, wherein the solution to be loaded on the cation exchange material has an electrical conductivity of 3 to 5 mS/cm.

25. The method according to any one of claims 1 to 24, wherein the cation exchange material pool is further processed through hydrophobic interaction chromatography to obtain a pool solution.

26. The method according to claim 25, wherein the hydrophobic interaction chromatography is performed in a flow-through mode.

27. The method according to claim 26, wherein the hydrophobic interaction chromatography is performed by use of a resin or a membrane.

28. The method according to claim 27, wherein the hydrophobic interaction chromatography resin or membrane has a phenyl group, a benzyl group or a hexyl group.

29. The method according to claim 28, wherein the hydrophobic interaction chromatography material is POROS Benzyl Ultra, POROS Benzyl, Hexyl-650C, Phenyl Sepharose 6 Fast Flow, Phenyl Sepharose High Performance, Sartobind Phenyl or ReadyToProcess Adsorber Phenyl.

30. The method according to any one of claims 1 to 14, wherein the activated carbon material pool is processed through hydrophobic interaction chromatography to obtain a pool solution, which is further processed through a cation exchange material to obtain a pool solution.

31. The method according to claim 30, wherein the hydrophobic interaction chromatography is performed in a flow-through mode.

32. The method according to claim 31, wherein the hydrophobic interaction chromatography is performed by use of a resin or a membrane.

33. The method according to claim 32, wherein the hydrophobic interaction chromatography resin or membrane has a phenyl group, a benzyl group or a hexyl group.

34. The method according to claim 33, wherein the hydrophobic interaction chromatography material is POROS Benzyl Ultra, POROS Benzyl, Hexyl-650C, Phenyl Sepharose 6 Fast Flow, Phenyl Sepharose High Performance, Sartobind Phenyl or ReadyToProcess Adsorber Phenyl.

35. The method according to any one of claims 30 to 34, wherein the hydrophobic interaction chromatography pool is loaded on the cation exchange material in an amount exceeding the binding capacity of the cation exchange material.

36. The method according to claim 35, wherein the hydrophobic interaction chromatography pool is loaded on the cation exchange material at a loading density of 500 to 2000 g/L.

37. The method according to claim 36, wherein the hydrophobic interaction chromatography pool is loaded on the cation exchange material at a loading density of 1000 to 1500 g/L.

38. The method according to any one of claims 30 to 37, wherein the cation exchange material is a resin, a monolith column or a membrane.

39. The method according to claim 38, wherein the cation exchange material has a functional group, which is sulfopropyl, sulfoethyl, sulfoisobutyl or carboxyl.

40. The method according to claim 39, wherein the cation exchange material is POROS XS, Eshmuno CPX, Eshmuno CP-FT or CaptoS ImpAct.

41. The method according to any one of claims 30 to 40, wherein the solution to be loaded on the cation exchange material has a pH of 4 to 6.

42. The method according to claim 41, wherein the solution to be loaded on the cation exchange material has a pH of 4.5 to 5.5.

43. The method according to any one of claims 30 to 42, wherein the solution to be loaded on the cation exchange material has an electrical conductivity of 3 to 7 mS/cm.

44. The method according to claim 43, wherein the solution to be loaded on the cation exchange material has an electrical conductivity of 3 to 5 mS/cm.

45. The method according to any one of claims 1 to 44, wherein at least one of the impurities is selected from the group consisting of host cell-derived proteins (host cell proteins; HCPs), phospholipase B-like 2 (PLBL2), viruses, virus-like particles, high-molecular weight species (HMWS), low-molecular weight species (LMWS), culture medium components, leached protein A and/or DNA.

46. The method according to any one of claims 1 to 45, wherein the steps are carried out continuously.

47. An antibody purified by a method according to any one of claims 1 to 46.

EP 3 916 001 A1

**INTERNATIONAL SEARCH REPORT**

| International application No. |
|---|
| PCT/JP2020/002057 |

A. CLASSIFICATION OF SUBJECT MATTER
C07K 1/16(2006.01)i; C07K 1/18(2006.01)i; C07K 1/22(2006.01)i; C07K 1/36(2006.01)i; C07K 16/00(2006.01)i; C12P 21/08(2006.01)i
FI: C07K1/16; C07K1/36; C07K1/18; C07K1/22; C07K16/00; C12P21/08
According to International Patent Classification (IPC) or to both national classification and IPC

B. FIELDS SEARCHED

Minimum documentation searched (classification system followed by classification symbols)
C07K1/16; C07K1/18; C07K1/22; C07K1/36; C07K16/00; C12P21/08

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | WO 2014/004281 A1 (EMD MILLIPORE CORPORATION) 03.01.2014 (2014-01-03) claims 3-4, 7, 25-26, 35, 45-46, paragraphs [0221]-[0233], examples 1-2, 7 | 1-3, 13-47 |
| Y | | 1-47 |
| X | JP 2013-189427 A (EMD MILLIPORE CORP.) 26.09.2013 (2013-09-26) claims 21-23, 25, paragraphs [0042], [0079], [0188], examples 9-12 | 1-4, 9, 13-47 |
| Y | | 1-47 |
| Y | WO 2014/004103 A1 (EMD MILLIPORE CORPORATION) 03.01.2014 (2014-01-03) claims 9, 13-20 | 1-47 |
| Y | WO 2018/043645 A1 (KYOWA HAKKO KIRIN CO., LTD.) 08.03.2018 (2018-03-08) claims 1-19, paragraphs [0012]-[0013], [0084] | 1-47 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 March 2020 (06.03.2020) | 17 March 2020 (17.03.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

31

INTERNATIONAL SEARCH REPORT

Information on patent family members

International application No.

PCT/JP2020/002057

| Patent Documents referred in the Report | Publication Date | Patent Family | Publication Date |
|---|---|---|---|
| WO 2014/004281 A1 | 03 Jan. 2014 | JP 2015-522019 A<br>claims 3-4, 7, 25-26, 35, 45-46, paragraphs [0199]-[0211], examples 1-2, 7<br>US 2015/0133636 A1<br>EP 2682168 A1<br>CN 104395341 A | |
| JP 2013-189427 A | 26 Sep. 2013 | US 2013/0245139 A1<br>claims 21-23, 25, examples 9-12<br>WO 2013/138098 A1<br>EP 2639239 A2<br>KR 10-2013-0105340 A<br>CN 103382215 A | |
| WO 2014/004103 A1 | 03 Jan. 2014 | JP 2015-522017 A<br>claims 9, 13-20<br>US 2015/0064769 A1<br>EP 2867359 A1<br>CN 104411820 A | |
| WO 2018/043645 A1 | 08 Mar. 2018 | US 2019/0211056 A<br>claims 1-19, paragraphs [0035]-[0036], [0174]<br>EP 3508493 A1 | |

**EP 3 916 001 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011150110 A
- WO 2013028330 A
- WO 2015038888 A
- WO 2015005961 A

**Non-patent literature cited in the description**

- **REICHERT, J.** Marketed therapeutic antibodies compendium. *MABs,* 2012, vol. 4 (3), 413-415
- **BROWN, A. ; BILL, J. ; TULLY, T. ; RADHAMO-HAN, A. ; DOWD, C.** Overloading ionexchange membranes as a purification step for monoclonal antibodies. *Biotechnol. Appl. Biochem.,* 2010, vol. 56, 59-70
- **LIU, H.F. ; MCCOOEY, B. ; DUARTE, T. ; MYERS, D.E. ; HUDSON, T. ; AMANULLAH, A. ; VAN REIS, R. ; KELLEY, B.D.** Exploration of overloaded cation exchange chromatography for monoclonal antibody purification. *J. Chromatogr. A.,* 2011, vol. 1218, 6943-6952
- **M. PAVLOVIC et al.** *Horm. Res.,* 2008, vol. 69, 14-21
- **T HALL et al.** *J. Chromatogr. A,* 2016, vol. 105, 1633-1642
- **B. TRAN et al.** *J. Chromatogr. A,* 2016, vol. 1438, 31-38